# EUROPEAN PATENT APPLICATION

(11) **EP 1 705 256 A1**
(43) Date of publication of application: **27.09.2006**
(21) Application number: 06251574.7
(22) Date of filing: 23.03.2006
(51) Int. Cl.: C12Q 1/68, G01N 33/50

(54) **Methods for the treatment of insulin resistance and disease states characterized by insulin resistance**

(30) Priority: 24.03.2005 US 664792 P
(71) Applicant: THE UAB RESEARCH FOUNDATION, Birmingham, AL 35294-0111 (US); F. Hoffmann-La Roche Ltd., 4070 Basel (CH)
(72) Inventor: Martin, Mitchell Lee, Verona, Essex County New Jersey 07044 (US); Garvey, W. Timothy, MD, Mountain Brook, AL 35223 (US); Fu, Yuchang, PhD, Mountain Brook, AL 35223 (US)
(74) Representative: Arends, William Gerrit

(57) **Abstract**

The MINOR and TR3 genes are disclosed to be insulin responsive genes differentially expressed as a function of insulin resistance and Type 2 Diabetes in humans and a variety of well characterized animal models. The present disclosure shows that MINOR and TR3 have a functional role in insulin resistance. Therefore, MINOR and TR3 are attractive and novel therapeutic targets for combating insulin resistance and/or disease states and conditions characterized by insulin resistance. Correlation of insulin resistance and disease states and conditions characterized by insulin resistance, including Type II diabetes, with MINOR and TR3 gene expression are also disclosed. Methods of diagnosis, treatment and prevention are likewise disclosed for the treatment and/or prevention of insulin resistance and disease states or conditions characterized by insulin resistance.

## Description

### FIELD OF THE DISCLOSURE

The present disclosure is directed to methods for the treatment and/or prevention of insulin resistance and disease states and conditions characterized by insulin resistance, and more particularly for the treatment and/or prevention of insulin resistance in skeletal muscles via novel drug targets, biomarkers and gene expression. Such methods may be used to treat a subject suffering from insulin resistance and a variety of disease states and conditions characterized by insulin resistance or to prevent the occurrence insulin resistance or disease states and conditions characterized by insulin resistance in an at risk subject.

### BACKGROUND

Diabetes is a disease caused by defects in insulin secretion and/or defects in a subject's response to the effects of insulin (i.e., insulin resistance), resulting in dysregulation of glucose metabolism. There are two major forms of diabetes: Type 1 diabetes and Type 2 diabetes. Type 1 diabetes is typically an autoimmune disease characterized by the loss of pancreatic β-cell function and an absolute or substantially absolute deficiency of insulin production. In the US, ~5% of patients with diabetes suffer from Type 1 diabetes. The remainder of the diabetic population (~95%) suffers from Type 2 diabetes, which is primarily due to defects in one or both of two physiological processes: 1) pancreatic insufficiency, a deficiency in secretion of insulin from the pancreas in response to rising blood glucose levels such as following a meal; and, 2) insulin resistance, the inability of target tissues of insulin action, primarily skeletal muscle, fat and liver, to respond to the hormone. The result of these defects is elevated blood glucose leading to glucose-mediated cellular toxicity and subsequent morbidity (nephropathy, neuropathy, retinopathy, etc.). Insulin resistance is strongly correlated with the development of Type 2 diabetes.

Insulin resistance is also associated with a number of disease states and conditions and is present in approximately 30-40% of non-diabetic individuals. These disease states and conditions include, but are not limited to, pre-diabetes and metabolic syndrome (also referred to as insulin resistance syndrome) (1-3). Pre-diabetes is a state of abnormal glucose tolerance characterized by either impaired glucose tolerance (IGT) or impaired fasting glucose (IFG). Patients with pre-diabetes are insulin resistant and are at high risk for future progression to overt Type 2 diabetes. Metabolic syndrome is an associated cluster of traits that include, but is not limited to, hyperinsulinemia, abnormal glucose tolerance, obesity, redistribution of fat to the abdominal or upper body compartment, hypertension, dysfibrinolysis, and a dyslipidemia characterized by high triglycerides, low HDL-cholesterol, and small dense LDL particles (1-4). Insulin resistance has been linked to each of the traits, suggesting metabolic syndrome and insulin resistance are intimately related to one another. The diagnosis of metabolic syndrome is a powerful risk factor for future development of Type 2 Diabetes, as well as accelerated atherosclerosis resulting in heart attacks, strokes, and peripheral vascular disease.

As skeletal muscle is the predominant target tissue for insulin-mediated glucose uptake (responsible for approximately 80-95% of glucose uptake), and is a critical locus of insulin resistance, defects in glucose uptake in skeletal muscle is a predominate contributor to the clinical manifestations of insulin resistance. The molecular basis for insulin resistance is not fully understood, but appears to involve defects in insulin signal transduction and abnormal cellular trafficking of glucose transporter proteins (5). The effect of insulin on gene expression in human muscle has not been extensively studied, and most prior studies focused on single genes or small numbers of genes with a limited focus (6-8).

Therefore, insulin resistance is a component in the pathogenesis of multiple human disease states and conditions including, but not limited to, metabolic syndrome, pre-diabetes, polycystic ovary syndrome, type 2 diabetes, dyslipidemia, obesity, infertility, inflammatory disorders, cancer, inflammatory diseases, Alzheimer's disease, hypertension, atherosclerosis, cardiovascular disease and peripheral vascular disease. Treatment modalities that combat insulin resistance can be used to effectively treat and prevent not only insulin resistance per se, but also disease states or conditions characterized by insulin resistance. Since in normoglycemic (normal glycemic) individuals skeletal muscle is responsible for approximately 80-95% of the glucose taken up from the blood in response to insulin, insulin resistance in skeletal muscle represents a primary target tissue for anti-diabetic therapies and key source of candidate drug targets.

### SUMMARY OF THE INVENTION

The present disclosure is directed to methods for the treatment and/or prevention of insulin resistance and the disease states and conditions characterized by insulin resistance, such as but not limited to Type II diabetes, and more particularly for the treatment and/or prevention of insulin resistance via novel drug targets, biomarkers and gene expression. The methods disclosed may be used to treat a subject suffering from insulin resistance and/or a variety of disease states and conditions characterized by insulin resistance. The methods disclosed may also be used to prevent the occurrence insulin resistance or disease states and conditions characterized by insulin resistance in an at risk subject.

The disclosure also demonstrates that the MINOR and TR3 genes are components of a general pathway that is involved in the insulin-mediated uptake of glucose from the blood. The present disclosure shows that insulin increases the expression of the MINOR and TR3 genes and further shows that increased expression of the MINOR gene enhances insulin-responsive glucose transport and enhances insulin-mediated recruitment of the GLUT-4 glucose transporters to the plasma membrane. The present disclosure also provides methods of diagnosing a susceptibility to insulin resistance and/or disease states and conditions characterized by insulin resistance in an individual in need of such diagnosis, such as for example Type II diabetes by detecting the activity and/or expression of the MINOR and/or TR3 gene and its concurrent pathway. The present disclosure also provides for methods to treat and/or prevent insulin resistance in a subject in need of such treatment or prevention by activating the MINOR and/or TR3 pathway. The present disclosure provides for methods to treat and/or prevent disease states and conditions characterized by insulin resistance in a subject in need of such treatment or prevention by activating the MINOR and/or TR3 pathway.

According to one aspect of the present invention, there is provided a method for treating or preventing insulin resistance in a subject, said method comprising identifying a subject in need of said treatment or prevention and activating an insulin responsive gene selected from the group consisting of MINOR, TR3 and MINOR and TR3.

According to another aspect of the present invention, there is provided a method for treating or preventing a disease state associated insulin resistance in a subject, said method comprising identifying a subject in need of said treatment or prevention and activating an insulin responsive gene selected from the group consisting of MINOR, TR3 and MINOR and TR3.

According to a further aspect of the present invention, there is provided a method for identifying a compound which activates an insulin responsive gene, said method comprising:
providing a cell expressing at least a portion of said insulin responsive gene, said insulin responsive gene selected from the group consisting of: MINOR, TR3 and MINOR and TR3;
incubating said cell with said compound and;
measuring a response to said compound.

According to another aspect of the present invention, there is provided a method for identifying a compound useful for the treatment or prevention of insulin resistance, said method comprising the step of determining whether the compound interacts with the polypeptide product of a gene selected from the group consisting of: MINOR, TR3 and MINOR and TR3.

According to a further aspect of the present invention, there is provided a method for identifying a compound useful for the treatment or prevention of insulin resistance, said method comprising the step of determining whether the compound activates a gene selected from the group consisting of: MINOR, TR3 and MINOR and TR3.

According to yet another aspect of the present invention, there is provided a method for identifying a compound useful for the treatment or prevention of a disease state associated with insulin resistance, said method comprising the step of determining whether the compound interacts with the polypeptide product of a gene selected from the group consisting of: MINOR, TR3 and MINOR and TR3.

According to a further aspect of the present invention, there is provided a method for identifying a compound useful for the treatment or prevention of a disease state associated with insulin resistance, said method comprising the step of determining whether the compound activates a gene selected from the group consisting of: MINOR, TR3 and MINOR and TR3.

### BRIEF DESCRIPTION OF THE FIGURES

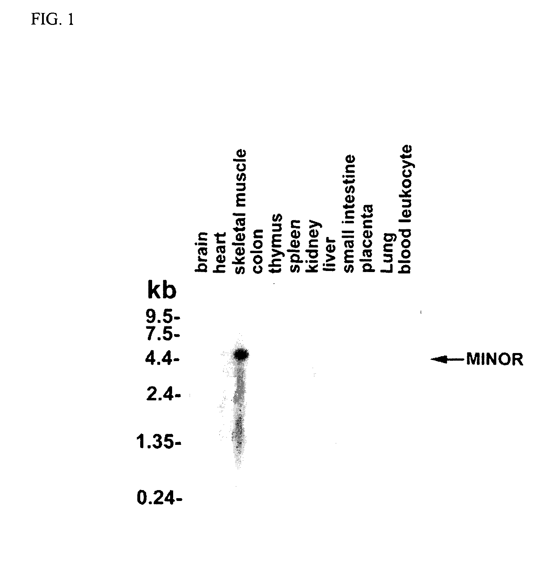

FIG. 1 shows MINOR gene expression in human tissues. Northern blot analysis was performed to examine MINOR gene expression in human tissues. Human multiple tissue northern blot was purchased from Clontech (Palo Alto, CA) and used as per manufacturer's instructions. Each of the lanes contained mRNA from the specific human tissue and the amount of each RNA blotted on the membrane was normalized with the β-actin cDNA control probe. The probe for detecting the MINOR gene hybridization signal was a 1.1 kb cDNA fragment corresponding to nucleotides 3874 to 4976 (17).

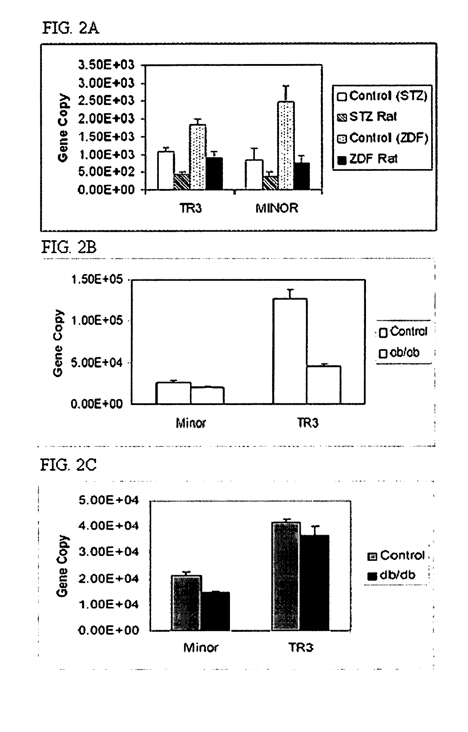

FIGS. 2A-C show MINOR and TR3 gene expression in skeletal muscles of diabetic and insulin resistant rats and mice. The skeletal muscle tissues from diabetic and insulin resistant rats or mice were homogenized and the mRNAs were extracted for cDNA synthesis. Quantitative real-time PCR was used to measure expression of the MINOR and TR3 genes. FIG. 2A shows a comparison of MINOR and TR3 gene expression between streptozotocin-induced diabetic rats (STZ Rat) and Zucker diabetic fatty rats (ZDF Rat).
FIG. 2B shows a comparison of MINOR and TR3 gene expression between ob/ob mice and control mice (ob/ob designates an insulin resistant mouse model in which a defect in the adipocyte-derived hormone leptin is expressed; control mice indicate mice that lack the deficit). FIG. 2C show a comparison of MINOR and TR3 gene expression between db/db mice and control mice (db/db designates an insulin resistant and diabetic mouse model in which a defect in the leptin receptor is expressed; control mice indicate mice that lack the deficit). All results represent the mean ± SE from three separate experiments.

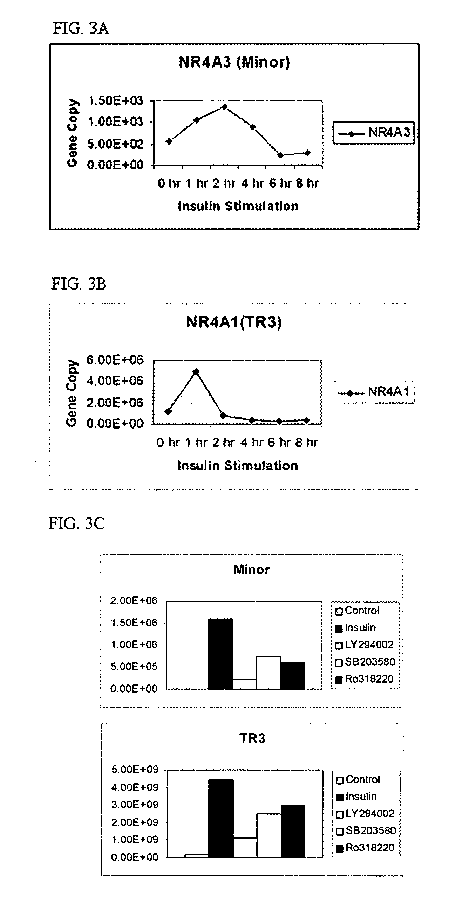

FIGS. 3A-3C show the effect of insulin stimulation on MINOR and TR3 gene expression in 3T3-L1 adipocytes. FIGS. 3A and 3B show insulin induces the expression of MINOR and TR3 gene expression in 3T3-L1 adipocytes. Fully differentiated 3T3-L1 adipocytes were treated with 100 nM of insulin for up to 8 hours (treated) or 0 nM insulin (0 hour control). The control and treated adipocytes were lysed and the mRNAs were extracted for cDNA synthesis. Quantitative real-time PCR was used to measure expression of MINOR (FIG. 3A) and TR3 (FIG. 3B) genes. Results represent the mean ± SE from three separate experiments. FIG. 3C shows inhibition of various signaling pathways decreases insulin-stimulated MINOR and TR3 gene expression. Fully differentiated 3T3-L1 adipocytes were treated with 100 nM of insulin alone (control) or plus LY294002 (PI 3-kinase inhibitor), SB203580 (p38 MAP kinase inhibitor), Ro318220 (protein kinase C inhibitor) for one hour. The control and treated adipocytes were lysed and the mRNAs were extracted for cDNAs synthesis. A quantitative real-time PCR was performed for detecting the expression levels of MINOR and TR3 genes. Results represent the mean ± SE from three separate experiments.

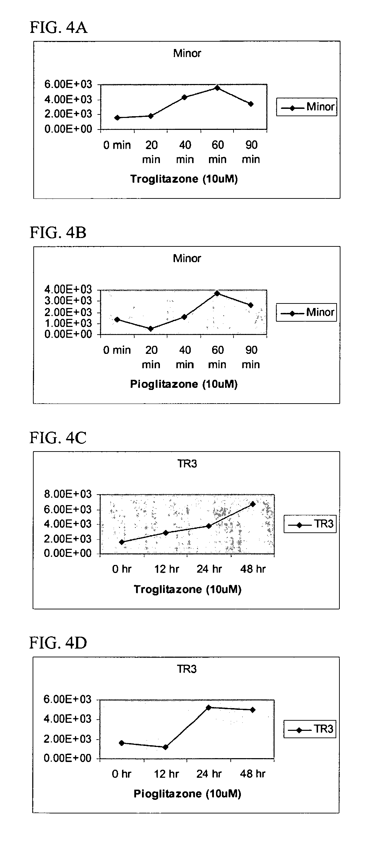

FIGS. 4A-4D shows thiazolidinediones (TZDs) stimulate MINOR and TR3 gene expression in 3T3-L1 adipocytes. Fully differentiated 3T3-L1 adipocytes were treated with 10 uM of the indicated thiazolidinedione (troglitazone or pioglizatone) from 0 to 48 hours. Control cells received vehicle alone. The control and treated adipocytes were lysed and the mRNAs were extracted for cDNA synthesis. Quantitative real-time PCR was used to measure expression levels of MINOR (FIGS. 4A and 4B) and TR3 (FIGS. 4C and 4D) genes. Results represent the mean ± SE from three separate experiments.

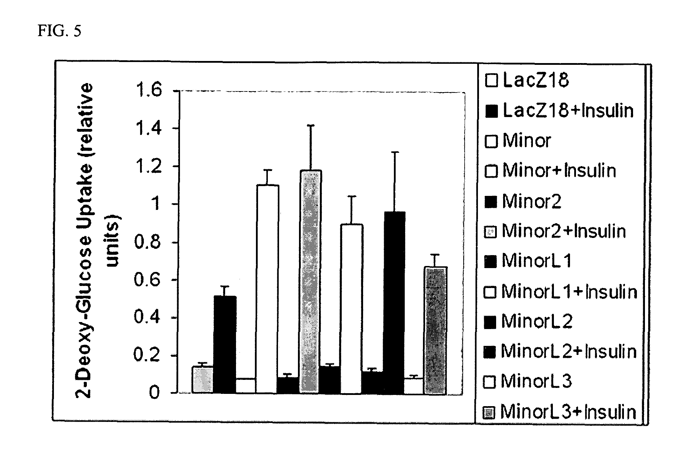

FIG. 5 shows MINOR enhances insulin-responsive glucose transport. Fully-differentiated adipocytes, a control adipocyte cell line hyperexpressing LacZ (LacZ 18), and five different MINOR hyperexpressing cell lines (Minor; Minor2; MinorL1; MinorL2; and MinorL3) were incubated in the absence (basal) and presence of insulin (100 nM) for 30 min at 37 °C. Measurements of 2-deoxy glucose transport were then performed (25). Results represent the mean ± SE from three separate experiments; p < 0.01 for comparing insulin-stimulated control and insulin stimulated MINOR hyperexpressing cells.

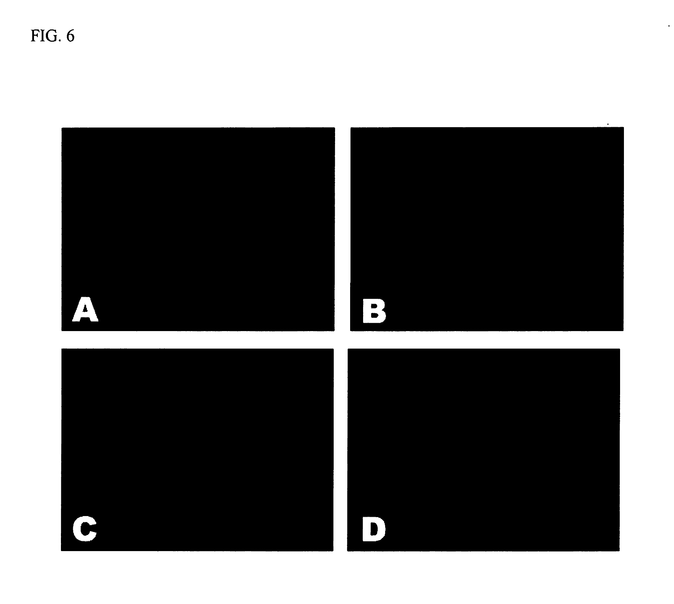

FIGS.6A-6D show the effect of MINOR gene expression on insulin-mediated recruitment of GLUT4 glucose transporters to plasma membrane. MINOR gene transduced 3T3-L1 fibroblasts and control LacZ transduced fibroblasts were grown on glass cover slips and differentiated into adipocytes. Adipocytes were then stimulated for 30 min with (insulin-stimulated) or without (basal) 100 nM of insulin. The adipocytes were then washed, and disrupted by sonication leaving and plasma membrane sheets attached to cover slips (plasma membrane lawn assay) (26). Plasma membrane associated GLUT4 was detected using a polyclonal anti-GLUT4 antibody and a FITC-conjugated secondary antibody. (A) basal LacZ expressing adipocytes; (B) insulin-stimulated LacZ expressing adipocytes; (C) basal Minor expressing adipocytes; (D) insulin-stimulated Minor expressing adipocytes.

### DETAILED DESCRIPTION

The present disclosure describes the discovery of two novel candidate targets for pharmaceutical intervention from an exhaustive genomic study of over 100 skeletal muscle biopsies from normal and insulin resistant human donors. By assaying gene expression profiles for over 12,000 human sequence probes (Affymetrix U95A) followed by multivariate analysis of over 50 detailed clinical parameters for each biopsy (eg, whole body glucose uptake assessed by hyperinsulinemic/euglycemic clamp), several hundred genes were identified with expression profiles which differ significantly in normal donors compared to insulin resistant individuals. Among this set are two genes (MINOR and TR3) in particular which, based on their protein sequence and presumed three-dimensional structure, are novel members of a sub-family of nuclear hormone receptors (NHRs) that include PPARγ, a known drug target of the thiozolidinediones (TZDs) class of insulin-sensitizing drugs (eg, troglitazone, rosiglitazone, pioglitazone etc.). Many NHRs are known to be 'master regulator' proteins which control entire programs of downstream gene expression with consequent effects on tissue physiology. The TZDs represent a new class of anti-diabetic compounds and the only existing drugs focusing on insulin resistance in skeletal muscle. Though generally efficacious, these compounds are not without unwanted side-effects such as inducing weight gain in some patients. The discovery of novel drug targets with related biophysical properties but distinct function thus offers significant therapeutic potential. Moreover, the expression profile of the protein products of the MINOR and/or TR3 genes described in this disclosure may represent a biomarker used to assess the degree of insulin resistance in an individual, either through direct assay of skeletal muscle or indirectly by measuring the amount of a surrogate biomarker in blood which is itself regulated directly or indirectly by the expression profile of these NHRs.

The present disclosure demonstrates that the MINOR and TR3 genes are insulin responsive genes and that the MINOR and TR3 genes are differentially expressed as a function of insulin resistance and Type 2 Diabetes in humans and a variety of well characterized animal models. Furthermore, the present disclosure shows that MINOR and TR3 have a functional role in increasing insulin sensitivity. Specifically, the present disclosure demonstrates that: 1) MINOR expression is limited to insulin target tissues, muscle and fat, while TR3 is also expressed in these tissues as well as being more ubiquitously expressed; 2) the expression of the MINOR and TR3 genes are consistently decreased in muscle from rodent models of insulin resistance, Type 2 Diabetes, and obesity; 3) MINOR and TR3 are induced by insulin in 3T3-L1 adipocytes via signal transmission through metabolic pathways used in insulin-mediated signal transduction (PI3-kinase, p38MAP kinase and PKC); 4) MINOR and TR3 are induced by thiazolidinedione insulin-sensitizing drugs in 3T3-L1 adipocytes; 5) in lentiviral vector stably-transduced adipocyte cell lines, MINOR expression markedly augments insulin sensitivity for stimulation of glucose transport and 6) increased MINOR expression leads to increased stimulation of glucose transport as a result of increased mobilization of the GLUT4 glucose transporter proteins to the cell surface of insulin-responsive cell types.

### Definitions

The terms "prevention", "prevent", "preventing", "suppression", "suppress" and "suppressing" as used herein refer to a course of action (such as administering a compound or pharmaceutical composition) initiated prior to the onset of a clinical symptom of a disease state or condition so as to prevent or reduce a clinical manifestation of the disease state or condition. Such preventing and suppressing need not be absolute to be useful.

The terms "treatment", "treat" and "treating" as used herein refers a course of action (such as administering a compound or pharmaceutical composition) initiated after the onset of a clinical symptom of a disease state or condition so as to eliminate or reduce a clinical manifestation of the disease state or condition. Such treating need not be absolute to be useful.

The term "in need of treatment" as used herein refers to a judgment made by a caregiver that a patient requires or will benefit from treatment. This judgment is made based on a variety of factors that are in the realm of a caregiver's expertise, but that includes the knowledge that the patient is ill, or will be ill, as the result of a condition that is treatable by a method or compound of the disclosure.

The term "in need of prevention" as used herein refers to a judgment made by a caregiver that a patient requires or will benefit from prevention. This judgment is made based on a variety of factors that are in the realm of a caregiver's expertise, but that includes the knowledge that the patient will be ill or may become ill, as the result of a condition that is preventable by a method or compound of the disclosure.

The term "individual", "subject" or "patient" as used herein refers to any animal, including mammals, such as mice, rats, other rodents, rabbits, dogs, cats, swine, cattle, sheep, horses, or primates, and humans. The term may specify male or female or both, or exclude male or female.

The term "therapeutically effective amount" as used herein refers to an amount of a compound, either alone or as a part of a pharmaceutical composition, that is capable of having any detectable, positive effect on any symptom, aspect, or characteristics of a disease state or condition. Such effect need not be absolute to be beneficial.

The term "insulin resistance" as used herein refers to a condition where a normal amount of insulin is unable to produce a normal physiological or molecular response. In some cases, a hyper-physiological amount of insulin (such as over 100 units), either endogenously produced or exogenously added, is able to overcome the insulin resistance in whole or in part and produce a biologic response.

### MINOR and TR3

The MINOR and TR3 genes are components of a general pathway that is involved in the insulin-mediated uptake of glucose from the blood. Consistent with this observation, the present disclosure shows that insulin increases the expression of the MINOR and TR3 genes and further shows that increased expression of the MINOR gene enhances insulin-responsive glucose transport and enhances insulin-mediated recruitment of the GLUT-4 glucose transporters to the plasma membrane. The present disclosure also provides for methods to treat and/or prevent insulin resistance in a subject in need of such treatment or prevention by activating the MINOR and/or TR3 pathway. The present disclosure provides for methods to treat or prevent disease states and conditions characterized by insulin resistance in a subject in need of such treatment or prevention by activating the MINOR and/or TR3 pathway. The disease states and conditions include, but are not limited to, metabolic syndrome, pre-diabetes, polycystic ovary syndrome, type 2 diabetes, dyslipidemia, obesity, infertility, inflammatory disorders, cancer, inflammatory diseases, Alzheimer's disease, hypertension, atherosclerosis, cardiovascular disease and peripheral vascular disease. Activating the MINOR and/or TR3 pathway includes, but is not limited to, increasing the absolute levels of the products of MINOR and/or TR3 gene expression, increasing the expression level or rate of the MINOR and/or TR3 genes, increasing the stability of the products of MINOR and/or TR3 gene expression, altering the levels and/or activity the polypeptide products of the MINOR and/or TR3 genes, increasing or decreasing the activity or expression of proteins downstream of MINOR and/or TR3 in the MINOR and/or TR3 pathway or a combination of any of the foregoing. Such activation of the MINOR and/or TR3 pathways may be achieved by direct or indirect methods. For example, direct methods may include, but are not limited to, providing increased levels of MINOR and/or TR3 gene expression, providing increased levels of the polypeptide products of MINOR and/or TR3 gene expression, or increasing the stability of the MINOR and/or TR3 genes or their expression products. Indirect methods may include, but are not limited to, activating downstream events in the MINOR and/or TR3 pathway. Such downstream events, include, but are not limited to, the stimulation or inhibition of proteins or enzymes that are influenced by MINOR and/or TR3. These proteins or enzymes may be involved in mediating the biological responses of the MINOR and/or TR3 pathway or antagonizing/down-regulating the biological responses of the MINOR and/or TR3 pathway. Any of the above may be accomplished through the administration of a pharmaceutical composition comprising at least one active ingredient or biologic or via the artificial induction of MINOR and/or TR3 genes or the expression products of such genes.

In one embodiment, the teachings of the present disclosure provide for the treatment of insulin resistance in a subject in need of such treatment. The method of treatment comprises the steps of identifying a subject in need of such treatment and activating the MINOR and/or TR3 gene pathway. In one embodiment, said activation is accomplished by increasing the expression of the MINOR and/or TR3 genes. In one embodiment, such increased expression is accomplished by administering a compound or pharmaceutical composition containing at least one active ingredient capable of activating the MINOR and/or TR3 pathway. In an alternate embodiment, such increased expression may be accomplished by introducing at least a portion of the MINOR and/or TR3 genes into a tissue (such as but not limited to skeletal muscle or adipose tissue) of said subject. Such activation would thereby treat insulin resistance in said subject. Such treatment may comprise increasing glucose uptake in insulin responsive tissues, increasing insulin sensitivity in insulin responsive tissues, or a combination of the foregoing. Other mechanisms may also be involved in such treatment.

In an alternate embodiment, the teachings of the present disclosure provide for the prevention of insulin resistance in a subject in need of such prevention. The method of prevention comprises the steps of identifying a subject in need of such prevention and activating the MINOR and/or TR3 gene pathway. In one embodiment, said activation is accomplished by increasing the expression of the MINOR and/or TR3 genes. In one embodiment, such increased expression is accomplished by administering a compound or pharmaceutical composition containing at least one active ingredient capable of activating the MINOR and/or TR3 pathway. In an alternate embodiment, such increased expression may be accomplished by introducing at least a portion of the MINOR and/or TR3 genes into a tissue (such as but not limited to skeletal muscle or adipose tissue) of said subject. Such activation would thereby prevent insulin resistance in said subject. Such prevention may comprise increasing glucose uptake in insulin responsive tissues, increasing insulin sensitivity in insulin responsive tissues, or a combination of the foregoing. Other mechanisms may also be involved in such prevention.

The present disclosure also provides for the treatment of disease states and conditions characterized by insulin resistance in a subject in need of such treatment by activating the MINOR and/or TR3 pathway. The method of treatment comprises the steps of identifying a subject in need of such treatment and activating the MINOR and/or TR3 gene pathway. In one embodiment, said activation is accomplished by increasing the expression of the MINOR and/or TR3 genes. In one embodiment, such increased expression is accomplished by administering a compound or pharmaceutical composition containing at least one active ingredient capable of activating the MINOR and/or TR3 pathway. In an alternate embodiment, such increased expression may be accomplished by introducing at least a portion of the MINOR and/or TR3 genes into a tissue (such as but not limited to skeletal muscle or adipose tissue) of said subject. Such activation would thereby treat at least one aspect of the disease state or condition in said subject. Such treatment may comprise increasing glucose uptake in insulin responsive tissues, increasing insulin sensitivity in insulin responsive tissues, or a combination of the foregoing. Other mechanisms may also be involved in such treatment.

The present disclosure also provides for the prevention of disease states and conditions characterized by insulin resistance in a subject in need of such treatment by activating the MINOR and/or TR3 pathway. The method of prevention comprises the steps of identifying a subject in need of such prevention and activating the MINOR and/or TR3 gene pathway. In one embodiment, said activation is accomplished by increasing the expression of the MINOR and/or TR3 genes. In one embodiment, such increased expression is accomplished by administering a compound or pharmaceutical composition containing at least one active ingredient capable of activating the MINOR and/or TR3 pathway. In an alternate embodiment, such activation may be accomplished by introducing at least a portion of the MINOR and/or TR3 genes into a tissue (such as but not limited to skeletal muscle or adipose tissue) of said subject. Such activation would thereby prevent at least one aspect of the disease state or condition in said subject. Such prevention may comprise increasing glucose uptake in insulin responsive tissues, increasing insulin sensitivity in insulin responsive tissues, or a combination of the foregoing. Other mechanisms may also be involved in such prevention

The methods of the treating and preventing discussed herein may also comprise further administering of one or more additional therapeutic agents agent in combination with those compounds or pharmaceutical compositions activating the MINOR and/or TR3 pathway. In one embodiment, the one or more additional therapeutic agents comprise metformin, sulforylurea or insulin.

Furthermore, the teachings of the present disclosure can be used to identify compounds that activate the MINOR and/or TR3 pathway. The compounds identified may thus be useful in the treatment and/or prevention methods described above. Such compounds may be small-molecule pharmaceuticals, peptides, biologics, various non-coding RNAs, antisense molecules and antibodies. The methods or assays for identifying such compounds comprise providing a cell line expressing at least a portion of the MINOR and/or TR3 genes, incubating said cells with a candidate compound and measuring a response to said candidate compound. Such a response may be any response that is measurable using analytical techniques currently known in the art. Exemplary responses include, but are not limited to, an increase in MINOR and/or TR3 gene expression levels, an increase in the level or activity of the polypeptides encoded by the MINOR and/or TR3 gene products or a functional response mediated by the MINOR and/or TR3 genes or their polypeptide products, such as increased glucose uptake, increased expression of a transporter involved in glucose regulation (such as, but not limited to, the GLUT4 transporter), increased translocation of a transporter involved in glucose regulation (such as, but not limited to, the GLUT4 transporter) to the cell membrane or increased activity of downstream signal transduction pathways regulated by MINOR and/or TR3 activity. Alternatively, cell lines may be provided that express downstream components activated in the MINOR and/or TR3 pathway and used in the methods of identification as discussed above. In addition, the response to said candidate compound measured may be a decrease in any of the criteria discussed above.

In an alternate embodiment, the teachings of the present disclosure can be used to identify compounds that bind to the expressed MINOR polypeptide, the expressed TR3 polypeptide or fragments of either polypeptide. The binding may be direct or indirect. By direct binding, it is meant the compound binds to the MINOR and/or TR3 polypeptide directly, without the involvement of an intermediate protein. By indirect binding, it is meant the compound binds to MINOR and/or TR3 via an intermediate polypeptide or structure, or the compound binds to a multi-protein complex comprising MINOR and/or TR3. The compounds identified may thus be useful in the treatment and/or prevention methods described above. Such compounds may be small-molecule pharmaceuticals, peptides, biologics, various non-coding RNAs, antisense molecules and antibodies. The methods or assays for identifying such compounds comprise providing a cell line expressing at least a portion of the MINOR and/or TR3 genes, incubating said cells with a candidate compound and measuring a determining whether said compound binds to the MINOR and/or TR3 polypeptides expressed in said cell line.

MINOR and/or TR3 genes, or fragments of these genes, may be also used utilizing the teachings of this invention for gene therapy by introducing the desired gene into the body of the patient who has or is suspected of having insulin resistance or a disease state or condition related to insulin resistance and is therefore in need of treatment and/or prevention. The desired gene may then be expressed and the treatment and/or prevention accomplished. Many methods exist for the introduction of genes or fragments thereof into a patient. For example, the gene may be introduced into a vector and introduced into the patient such that the gene or fragment thereof is expressed and the therapeutic potential realized. Exemplary methods of introduction include, but are not limited to, viral vectors (including retroviruses) and liposomes. Vectors may be introduced into a patient either *in vivo* or *ex vivo.* In the case of an in vivo treatment, the vector may be simply injected into the patient, for example parenterally, and allowed to find suitable target cells for gene expression. In the case of ex vivo treatment, cells are grown *in vitro* and transduced or transfected with the virus, embedded in a carrier such as a collagen matrix, which is then implanted in the patient, for example as a sub-cutaneous implant.

### Pharmaceutical Compositions

The compound or pharmaceutical composition for use in the methods described may be formulated by any method known in the art. Certain exemplary methods for preparing the compounds and pharmaceutical compositions are described herein and should not be considered as limiting examples. Furthermore, the compounds or pharmaceutical compositions may be administered to the subject as is known in the art and determined by a healthcare provider. Certain modes of administration are provided herein and should not be considered as limiting examples. Furthermore, the compound or pharmaceutical composition may be administered with other agents in the methods described herein. Such other agents may be agents that increase the activity of the compounds disclosed, such as by limiting the degradation or inactivation of the compounds disclosed or increasing the absorption or activity of the compounds disclosed.

The compounds and pharmaceutical compositions described can be used in the form of a medicinal preparation, for example, in aerosol, solid, semi-solid or liquid form which contains the compounds disclosed as an active ingredient. In addition, the pharmaceutical compositions may be used in an admixture with an appropriate pharmaceutically acceptable carriers. Such pharmaceutically acceptable carriers include, but are not limited to, organic or inorganic carriers, excipients or diluents suitable for pharmaceutical applications. The active ingredient may be compounded, for example, with the usual non-toxic pharmaceutically acceptable carriers, excipients or diluents for tablets, pellets, capsules, inhalants, suppositories, solutions, emulsions, suspensions, aerosols and any other form suitable for use. Pharmaceutically acceptable carriers for use in pharmaceutical compositions are well known in the pharmaceutical field, and are described, for example, in Remington: The Science and Practice of Pharmacy Pharmaceutical Sciences, Lippincott Williams and Wilkins (A. R. Gennaro editor, 20th edition). Such materials are nontoxic to the recipients at the dosages and concentrations employed and include, but are not limited to, water, talc, gum acacia, gelatin, magnesium trisilicate, keratin, colloidal silica, urea, buffers such as phosphate, citrate, acetate and other organic acid salts, antioxidants such as ascorbic acid, low molecular weight (less than about ten residues) peptides such as polyarginine, proteins, such as serum albumin, gelatin, or immunoglobulins, hydrophilic polymers such as polyvinylpyrrolidinone, amino acids such as glycine, glutamic acid, aspartic acid, or arginine, monosaccharides, disaccharides, and other carbohydrates including cellulose or its derivatives, lactose, mannitol, glucose, mannose, dextrins, potato or corn starch or starch paste, chelating agents such as EDTA, sugar alcohols such as mannitol or sorbitol, counterions such as sodium and/or nonionic surfactants such as Tween, Pluronics or polyethyleneglycol. In addition, the pharmaceutical compositions may comprise auxiliary agents, such as, but not limited to, taste-enhancing agents, stabilizing agents, thickening agents, coloring agents and perfumes.

Pharmaceutical compositions may be prepared for storage or administration by mixing a compound of the present disclosure having a desired degree of purity with physiologically acceptable carriers, excipients, stabilizers, auxiliary agents etc. as is known in the pharmaceutical field. Such pharmaceutical compositions may be provided in sustained release or timed release formulations.

The pharmaceutical compositions may be administered orally in solid dosage forms, such as capsules, tablets, and powders, or in liquid dosage forms, such as elixirs, syrups and suspensions. It can also be administered parenterally, in sterile liquid dosage forms. Furthermore, pharmaceutical compositions may be administered parenterally by transmucosal delivery via solid, liquid or aerosol forms of transdermally via a patch mechanism or ointment. Various types of transmucosal administration include respiratory tract mucosal administration, nasal mucosal administration, oral transmucosal (such as sublingual and buccal) administration and rectal transmucosal administration.

For preparing solid compositions such as, but not limited to, tablets or capsules, the pharmaceutical compositions may be mixed with an appropriate pharmaceutically acceptable carriers, such as conventional tableting ingredients (lactose, sucrose, mannitol, corn starch, potato starch, alginic acid, microcrystalline cellulose, acacia, gelatin, gums, colloidal silicon dioxide, croscarmellose sodium, talc, sorbitol, stearic acid magnesium stearate, calcium stearate, zinc stearate, stearic acid, dicalcium phosphate other excipients, colorants, diluents, buffering agents, disintegrating agents, moistening agents, preservatives, flavoring agents, and pharmacologically compatible carriers) and diluents (including, but not limited to, water, saline or buffering solutions) to form a substantially homogenous composition. The substantially homogenous composition means the components (a compound as described herein and a pharmaceutically acceptable carrier) are dispersed evenly throughout the composition so that the composition may be readily subdivided into equally effective unit dosage forms such as tablets, pills and capsules. The solid compositions described may be coated or otherwise compounded to provide a dosage form affording the advantage of prolonged action. For example, the tablet or pill can comprise an inner dosage an outer dosage component, the latter being in the form of an envelope over the former. The two components can be separated by an enteric layer which serves to resist disintegration in the stomach and permits the inner component to pass intact through the stomach or to be delayed in release. A variety of materials can be used for such enteric layers or coatings such materials including a number of polymeric acids and mixtures of polymeric acids with such materials as shellac, cetyl alcohol and cellulose acetate. The active compounds may also be formulated in rectal compositions such as suppositories or retention enemas, e.g., containing conventional suppository bases such as cocoa butter or other glycerides. The solid compositions may also comprise a capsule, such as hard- or soft-shelled gelatin type containing, for example, surfactants, lubricants, and inert fillers, such as lactose, sucrose, calcium phosphate, and corn starch.

For intranasal administration, intrapulmonary administration or administration by other modes of inhalation, the pharmaceutical compositions may be delivered in the form of a solution or suspension from a pump spray container or as an aerosol spray presentation from a pressurized container or nebulizer, with the use of a suitable propellant (e.g., dichlorodifluoromethane, trichlorofluoromethane, dichlorotetrafluoroethane, nitrogen, propane, carbon dioxide or other suitable gas) or as a dry powder. In the case of an aerosol or dry powder format, the amount (dose) of the compound delivered may be determined by providing a valve to deliver a metered amount.

Liquid forms may be administered orally, parenterally or via transmucosal administration. Suitable forms for liquid administration include aqueous solutions, suitably flavored syrups, aqueous or oil suspensions, and flavored emulsions with edible oils such as cottonseed oil, sesame oil, coconut oil, or peanut oil as well as elixirs and similar pharmaceutical vehicles. Suitable dispersing or suspending agents for aqueous suspensions include synthetic natural gums, such as tragacanth, acacia, alginate, dextran, sodium carboxymethyl cellulose, methylcellulose, polyvinylpyrrolidone or gelatin. Such liquid preparations may be prepared by conventional means with pharmaceutically acceptable additives such as suspending agents (e.g., sorbitol syrup, methyl cellulose or hydrogenated edible fats); emulsifying agents (e.g., lecithin or acacia); non-aqueous vehicles (e.g., almond oil, oily esters or ethyl alcohol); preservatives (e.g., methyl or propyl p-hydroxybenzoates or sorbic acid); and artificial or natural colors and/or sweeteners. Liquid formulations may include diluents, such as water and alcohols, for example, ethanol, benzyl alcohol, propylene glycol, glycerin, and the polyethylene alcohols, either with or without the addition of a pharmaceutically acceptable surfactant, suspending agent, or emulsifying agent. For buccal or sublingual administration, the composition may take the form of tablets or lozenges formulated in conventional manners. Lozenge forms can comprise the active ingredient in a flavor, usually sucrose and acacia or tragacanth, as well as pastilles comprising the active ingredient in an inert base, such as gelatin and glycerin, or sucrose and acadia, emulsions, and gels containing, in addition to the active ingredient, such carriers as are known in the art.

The compounds disclosed (whether alone or in pharmaceutical compositions) may be formulated for parenteral administration. Parenteral administration includes, but is not limited to, intravenous administration, subcutaneous administration, intramuscular administration, intradermal administration, intrathecal administration, intraarticular administration, intracardiac administration, retrobulbar administration and administration via implants, such as sustained release implants.

The pharmaceutical compositions may be presented in unit-dose or multi-dose sealed containers, such as ampules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets. The requirements for effective pharmaceutically acceptable carriers for injectable compositions are well known to those of ordinary skill in the art. See Pharmaceutics and Pharmacy Practice, J.B. Lippincott Co., Philadelphia, Pa., Banker and Chalmers, Eds., 238-250 (1982) and ASHP Handbook on Injectable Drugs, Toissel, 4th ed., 622-630 (1986).

The pharmaceutical compositions are administered in pharmaceutically effective amount. The pharmaceutically effective amount will, of course, vary depending upon known factors, such as the pharmacodynamic characteristics of the particular compound and its mode and route of administration; the age, health and weight of the subject; the severity and stage of the disease state or condition; the kind of concurrent treatment; the frequency of treatment; and the effect desired. The total amount of the compound administered will also be determined by the route, timing and frequency of administration as well as the existence, nature, and extent of any adverse side effects that might accompany the administration of the compound and the desired physiological effect. It will be appreciated by one skilled in the art that various conditions or disease states, in particular chronic conditions or disease states, may require prolonged treatment involving multiple administrations.

### EXAMPLES

### MINOR and TR3 Genes are Differentially Expressed in Insulin Resistance

The primary target tissues for insulin action are skeletal muscle, adipose tissue, and the liver tissue. Skeletal muscle is the predominant target tissue for insulin-mediated glucose uptake (responsible for approximately 95% of glucose uptake), and is a critical locus of insulin resistance. Defects in glucose uptake in skeletal muscle is a predominate contributor to the clinical manifestations of insulin resistance. The molecular basis for insulin resistance is not well understood, but appears to involve defects in insulin signal transduction and abnormal cellular trafficking of glucose transporter proteins (5). To better understand the molecular defects responsible for human insulin resistance, skeletal muscle biopsies were obtained from volunteers in three metabolically-defined subgroups: A) insulin sensitive: (B) insulin resistant; and (C) type 2 diabetic. Biopsies from each subgroup were obtained under both basal conditions and after three hours of hyperinsulinemia. Differential gene expression in these muscle biopsies was assessed using cDNA microarray technology utilizing the Affymetrix Hu95A gene expression chips (carried out as per manufacturer's instructions). The effect of insulin on gene expression in human muscle has not been extensively studied, and most prior studies focused on single genes or small numbers of genes with a limited focus (6-8).

We found that insulin resistance in subjects with or without Type 2 diabetes is associated with differential gene expression in skeletal muscle when compared with insulin sensitive individuals. In particular, over 100 genes coding for transcription factors were shown to be acutely regulated by insulin, and/or differentially expressed in skeletal muscle among the three defined subgroups. Many of these transcription factors expressed the zinc finger motif. Table 1 shows data (i.e., p values for statistically significant differential expression) for two of these genes including MINOR (Mitogen-Inducible Nuclear Orphan Receptor; GenBank accession number: U12767; SEQ ID NO. 1 shows the nucleotide sequence of the MINOR gene, designating the CDS; SEQ ID NO. 2 shows the polypeptide sequence of the peptide encoded by the MINOR gene) and TR3 (GenBank accession number: L13740; SEQ ID NO. 3 shows the nucleotide sequence of the TR3 gene, designating the CDS; SEQ ID NO. 4 shows the polypeptide sequence of the peptide encoded by the TR3 gene). Both MINOR and TR3 belong to the NGFI-B family of orphan nuclear receptors.

MINOR (also known as NOR-1, TEC, and CHN and designated NR4A3 in the nuclear receptor nomenclature system) is an orphan nuclear receptor originally identified as a protein induced in primary cultures of rat embryonic forebrain neurons undergoing apoptosis (9). Homology analysis of its DNA binding domain identifies MINOR as a member of the NGFI-B family of orphan nuclear receptors together with Nurr1 (also known as TINUR, NOT, and designated NR4A2 in the nuclear receptor nomenclature system) and TR3 (also known as Nur77, NGFI-B, and designated NR4A1 in the nuclear receptor nomenclature system). The NGFI-B receptors are members of steroid/thyroid receptor superfamily.

Among receptors in the NGFI-B family, homologies in the N-terminal transactivation domains and the C-terminal "ligand binding domains" are 37-53% and 53-77%, respectively. In the absence of any ligand, MINOR, Nurr1 and TR3 can each bind and activate the NGFI-B-responsive DNA element characterized by the nucleotide sequence AAAGGTCA (10). MINOR, Nurr1 and TR3 each share greater that 97% homology in their DNA binding domains, which consist of two zinc fingers and a domain termed the A box. In the presence of retinoic acid, however, TR3 and Nurr1, but not MINOR, can heterodimerize with the retinoid X receptor and regulate a DNA element composed of direct repeats separated by five nucleotides (DR5) (11-13). The NGFI-B proteins are immediate early gene products which are involved in neuroendocrine regulation, neural differentiation, liver regeneration, cell apoptosis, and mitogenic stimulation in different cell types (14-17).

### Tissue Specific Expression

To determine whether MINOR and/or TR3 regulation plays a role in insulin resistance, the tissue specific expression of MINOR and TR3 genes was examined. Northern blot analysis was performed to examine MINOR and TR3 gene expression in brain, heart, skeletal muscle, colon, thymus, spleen, kidney, liver, small intestine, placenta, lung and blood leukocytes (FIG. 1). Human multiple tissue northern blot was purchased from Clontech (Palo Alto, CA). Each of the lanes contained mRNA from the designated human tissue and the amount of each RNA blotted on the membrane was normalized with a β-actin cDNA control probe. The probe for detecting MINOR gene hybridization was a 1.1 kb cDNA fragment (17). The results show that MINOR was highly expressed only in human skeletal muscle and adipocytes, both of which comprise classic insulin target tissues. In contrast, TR3 was expressed in skeletal muscle and adipocytes, as well as multiple other tissues (data not shown).

### MINOR and TR3 expression are reduced in skeletal muscles of diabetic rats and mice

In order to determine whether the MINOR and/or TR3 genes are abnormally regulated in insulin resistance and/or Type 2 Diabetes, MINOR and TR3 gene expression was analyzed in several animal models. Zucker diabetic fatty (ZDF) rats, streptozotocin (STZ)-induced diabetic rats, db/db mice (mice that express defects in leptin receptor), and ob/ob mice (mice that express defects in the adipocyte-derived hormone leptin) and are well characterized as insulin-resistant, diabetic or obese animal models (18, 20). Skeletal muscle tissue from these diabetic and insulin resistant rats or mice was obtained, homogenized and the mRNA extracted for cDNA synthesis. The cDNA was used in quantitative real-time PCR to measure the expression of MINOR and TR3 genes.

The expression of MINOR and TR3 genes in skeletal muscle samples showed a decrease in MINOR and TR3 gene expression in these animal models of insulin resistance, diabetes and obesity as compared to the appropriate control animals. FIG. 2A shows that MINOR and TR3 gene expression was significantly decreased in the STZ and ZDF rat models. FIGS. 2B and 2C show similar results for the ob/ob and db/db mice models. Therefore, decreased expression of the MINOR and TR3 genes, which code for two transcription factors, is consistently associated with insulin resistance, diabetes and obesity in animal models. This result correlates with the decreased expression of MINOR and TR3 genes observed in skeletal muscle biopsies from insulin resistant and type-2 diabetic human subjects (Table 1).

### The expression of MINOR and TR3 in 3T3-L1 adipocytes

Since MINOR and TR3 gene expression was strongly induced by insulin stimulation in human skeletal muscles in the microarray analysis described above (Table 1), the responsiveness of MINOR and TR3 gene expression to insulin stimulation was examined in adipocytes, another major insulin target tissue. Fully differentiated 3T3-L1 adipocytes were treated with 100 nM of insulin for various times (0 to 8 hours; the 0 hour time point served as the control and received no added insulin). The control and treated adipocytes were lysed and the mRNAs were extracted for cDNAs synthesis. A quantitative real-time PCR was performed for detecting the expression levels of MINOR and TR3 genes. As shown in FIGS. 3A and 3B, insulin stimulated MINOR and TR3 gene expression (Nurr1 gene expression was not stimulated; data not shown) in fully differentiated adipocytes. FIG. 3A shows the effect of insulin on MINOR gene expression in adipocytes and shows a rapid increase in MINOR gene expression that is sustained over a 4 hour period. Insulin also stimulated TR3 gene expression adipocytes, but with a different temporal response with TR3 expression levels returning to baseline by hour 2 after treatment (FIG. 3B).

The stimulatory effect of insulin on MINOR and TR3 gene expression was dependent on signal transduction via the PI-3-kinase pathway, the p38 MAP-K pathway and the protein kinase C (PKC) pathway. Blocking any of these pathways by administration of specific chemical inhibitors decreased the ability of insulin to stimulate MINOR or TR3 gene expression (FIG. 3C). Fully differentiated 3T3-L1 adipocytes were treated for 1 hour with 100 nM of insulin alone or 100 nM of insulin plus LY294002 (PI 3-kinase inhibitor, 10 nM), SB203580 (p38 MAP kinase inhibitor, 10 nM), or Ro318220 (protein kinase C inhibitor, 50 nM). Control adipocytes received no insulin or inhibitors. The control and treated adipocytes were lysed and the mRNAs were extracted for cDNAs synthesis. A quantitative real-time PCR was performed for detecting the expression levels of MINOR and TR3 genes. As can be seen in FIG. 3C, inhibiting the PI-3-kinase pathway, the p38 MAP-K pathway or the protein kinase C (PKC) pathway inhibited the ability of insulin to stimulate MINOR and TR3 gene expression. Importantly, each of these pathways are implicated in the ability of insulin to stimulate the regulation and cycling of the GLUT4 glucose transporter. These results show that MINOR and TR3 are insulin responsive genes and are expressed in a specific target of insulin action (i.e. adipocytes). Furthermore, the results show that the insulin responsive increase in gene expression can be blocked, at least partially, with well characterized inhibitors of the insulin signal transduction cascade.

### Thiazolidinediones (TZDs) induce MINOR and TR3 in 3T3-L1 adipocytes

Next, the effect of several drugs in the thiazolidinedione class of insulin sensitizing drugs, namely troglitazone and pioglitazone, were examined for their ability to induce MINOR and TR3 gene expression in 3T3-L1 adipocytes. The thiazolidinediones (TZD) and thiazolidinedione-like drugs are known to improve insulin resistance and are used as a treatment for diabetes in animal models and patients (22). These drugs reduce plasma glucose and concomitantly lower hyperinsulinemia by improving the stimulation of muscle glucose uptake and the inhibition of hepatic glucose production by insulin. The antidiabetic actions of TZDs and TZD-like drugs are due to the activation of the peroxisome proliferator-activated receptor gamma (PPARγ) (23), a member of the nuclear receptor superfamily of transcription factors (24). TZD agonists of PPARγ have an effect on glucose disposal which is typically thought to occur in the muscle, however PPARγ is expressed in very small amounts in this tissue. Given the results that MINOR and TR3 are highly expressed in skeletal muscle, it is reasonable to speculate that TZDs could stimulate MINOR and TR3 gene expression. Alternatively, activation of MINOR and TR3 could represent downstream consequences of PPARγ activation, and constitute direct mediators of the insulin-sensitizing TZD drug effect.

The insulin sensitizing effects of TZDs and TZD-like drugs are accompanied by unwanted side effects such as weight gain, edema, intravascular volume expansion, anemia, and congestive heart failure. It remains possible that drugs acting downstream of PPARγ activation could lead to increased insulin sensitivity without the unwanted side effects of TZDs.

Fully differentiated 3T3-L1 adipocytes were treated with 10 uM of the indicated thiazolidinedione (troglitazone or pioglizatone) from 0 to 48 hours. Control cells (0 hour time point) received vehicle alone. The control and treated adipocytes were lysed and the mRNAs were extracted for cDNA synthesis. Quantitative real-time PCR was used to measure expression levels of MINOR (FIGS. 4A and 4B) and TR3 (FIGS. 4C and 4D) genes. FIGS. 4A-D show that troglitazone and pioglitazone stimulate MINOR and TR3 gene expression in adipocyte cells. Expression of these genes was unaffected in control cells over these time courses. Therefore, MINOR and TR3 were induced by thiazolidinedione drugs, which are used to increase insulin sensitivity in humans.

### MINOR enhances insulin action on glucose uptake in 3T3-L1 adipocytes

To identify the biological or functional effects of increased MINOR gene expression, recombinant MINOR lentivirus vectors were generated and used to stably transduce 3T3-L1 adipocyte cell lines. Several MINOR lentiviral vectors were generated. The MINOR cDNA sequences used are designated as follows: (i) MINOR; (ii) MINOR 2; (iii) MINOR L1; (iv) MINOR L2; and (v) MINOR L3. These constructs contained full-length MINOR coding sequences as follows: full CDS for MINOR; nucleotides 183 to 1971 of the CDS for MINOR 2; nucleotides 100 to 1971 of the CDS for MINOR L1, MINOR L2 and MINOR L3 (17). In each case, fusion cDNAs were created through the addition of a V5 epitope tag, and were cloned into a ViraPower-CMV vector (Invitrogen). The recombinant lentiviral plasmids and a control lentiviral LacZ gene construct were transfected into HEK293 cells to generate the recombinant lentiviruses. X-gal staining was performed to confirm that the HEK293 cell transfection was successful and that infectious virus particles were produced.

To establish stable 3T3-L1adipocyte cell lines which overexpress the MINOR or LacZ genes, recombinant MINOR or LacZ lentiviral stocks purified from HEK293 cells were used to infect 3T3-L1 adipocytes. Forty-eight hours post-transduction, these cells were placed under blasticidin selection (10 µg/ml) for 20 days. The tests for stable recombinant MINOR or LacZ gene expression were performed after antibiotic selection by Western blot analyses. These experiments yielded multiple clonal adipocyte cell lines with sustained expression of the described MINOR gene constructs the or LacZ gene.

Glucose uptake is the rate-limiting step in insulin's ability to stimulate glucose uptake and metabolism. Insulin augments the transport of glucose into target cells by increasing the concentration of a specific glucose transporter isoform, GLUT4, at the cell surface (5, 21). If MINOR is involved in the regulation of GLUT4, then MINOR transduced adipocytes should display increased insulin responsiveness for stimulation of the glucose transport system. As shown in FIG. 5, insulin's ability to maximally stimulate glucose uptake in fully differentiated adipocytes was increased over 80% (P < 0.01) in the MINOR overexpressing cells when compared with control LacZ overexpressing adipocytes. This was true for all 5 stably transduced MINOR-expressing cell lines. In full-dose response curves, glucose transport responses were markedly enhanced over the full range of insulin concentrations in MINOR-transduced cells, without changes in the insulin ED₅₀ for glucose transport stimulation (data not shown).

FIGS. 6A-6D show that MINOR gene expression increases the ability of insulin stimulation to recruit the GLUT-4 glucose transporter to the plasma membrane of adipocyte cells (SEQ ID NO. 5 shows the nucleotide sequence of the mouse GLUT4 gene, designating the CDS; SEQ ID NO. 6 shows the polypeptide sequence of the peptide encoded by the mouse GLUT4 gene; for reference purposes SEQ ID NO. 7 shows the nucleotide sequence of the human GLUT4 gene, designating the CDS; and SEQ ID NO. 8 shows the polypeptide sequence of the peptide encoded by the human GLUT4 gene). These experiments use the "plasma membrane lawn" technique (26) to quantify the relative amounts of GLUT4 glucose transporter proteins in the cell surface plasma membrane. MINOR gene transduced 3T3-L1 fibroblasts and control LacZ transduced fibroblasts were grown on glass cover slips and differentiated into adipocytes. In these experiments, the full-length MINOR constructs were used (similar results were obtained for the vectors containing MINOR nucleotides 183-1971 and 100-1971). Adipocytes were then stimulated for 30 min with or without (basal) 100 nM of insulin. The adipocytes were then washed, and disrupted by sonication leaving and plasma membrane sheets attached to cover slips. Plasma membrane associated GLUT4 was detected using a polyclonal anti-GLUT4 antibody and a FITC-conjugated secondary antibody.

In control (LacZ transfected) adipocytes, insulin stimulation leads to increased GLUT4 staining in the plasma membrane, consistent with the known ability of insulin to recruit increased numbers of intracellular GLUT4 transporters to the cell surface. In adipocytes hyperexpressing the MINOR gene, the ability of insulin stimulation to recruit GLUT4 glucose transporters to the plasma membrane is clearly stimulated as compared to control LacZ expressing adipocytes (compare FIGS. 6A and 6B to FIGS 6C and 6D). Therefore, MINOR gene expression increases insulin-stimulated glucose transport by enhancing the normal cellular processes that mediate the ability of insulin stimulation to transport glucose from the blood and into tissue.

### Discussion of Examples

The above examples demonstrate that the MINOR and TR3 genes are insulin responsive genes and that the MINOR and TR3 genes are differentially expressed as a function of insulin resistance and Type 2 Diabetes in humans and a variety of well characterized animal models. Furthermore, the present disclosure shows that MINOR and TR3 have a functional role in increasing insulin sensitivity. Specifically, theexamples demonstrate that: 1) MINOR expression is limited to insulin target tissues, muscle and fat, while TR3 is also expressed in these tissues as well as being more ubiquitously expressed; 2) the expression of the MINOR and TR3 genes are consistently decreased in muscle from rodent models of insulin resistance, Type 2 Diabetes, and obesity; 3) MINOR and TR3 are induced by insulin in 3T3-L1 adipocytes via signal transmission through metabolic pathways used in insulin-mediated signal transduction (PI3-kinase, p38MAP kinase and PKC); 4) MINOR and TR3 are induced by thiazolidinedione insulin-sensitizing drugs in 3T3-L1 adipocytes; 5) in lentiviral vector stably-transduced adipocyte cell lines, MINOR expression markedly augments insulin sensitivity for stimulation of glucose transport and 6) increased MINOR expression leads to increased stimulation of glucose transport as a result of increased mobilization of the GLUT4 glucose transporter proteins to the cell surface of insulin-responsive cell types.

These results demonstrate that insulin resistance is associated with reduced expression of MINOR and TR3 genes. Furthermore, the present disclosure demonstrates that the stimulation of MINOR and TR3 gene expression can produce a marked decrease insulin resistance and a corresponding increase in insulin sensitivity by enhancing the insulin-mediated clearance of glucose from the blood.

Therefore, MINOR and TR3 are attractive and novel therapeutic targets for combating insulin resistance. Specifically, activation of MINOR and TR3 pathway by small-molecule pharmaceuticals, by genetic manipulation, or by other means represents a new and effective approach to the treatment of insulin resistance or disease states or conditions characterized by insulin resistance. Furthermore, activation of downstream signaling events mediated by the MINOR and/or TR3 pathway is also a new and effective approach to the treatment of insulin resistance or disease states or conditions characterized by insulin resistance. These disease states and conditions include, but are not limited to, metabolic syndrome, pre-diabetes, polycystic ovary syndrome, type 2 diabetes, dyslipidemia, obesity, infertility, inflammatory disorders, cancer, inflammatory diseases, Alzheimer's disease, hypertension, atherosclerosis, cardiovascular disease and peripheral vascular disease.

As disclosed, in addition to constituting new direct therapeutic targets for the treatment and prevention of insulin resistance and the disease states and conditions characterized by insulin resistance, MINOR and TR3 can be also utilized for screening new therapeutic agents for treating insulin resistance and disease states and conditions characterized by insulin resistance. Such new therapeutic agents include, but are not limited to, synthetic or endogenous ligands for MINOR and TR3. Similarly, MINOR and TR3 could provide the basis for identifying downstream molecular events that mediate desirable therapeutic effects. For example, a search for genes regulated by MINOR and TR3, or factors that interact with the corresponding encoded proteins, could identify new drug targets or therapeutic approaches. The advances in the understanding of the molecular pathways that underlie MINOR or TR3 expression, gene regulation by these transcription factors, and related cellular effects could potentially point to a number of new opportunities for therapeutic intervention.

The foregoing description illustrates and describes the compounds of the present disclosure. Additionally, the disclosure shows and describes only certain embodiments of the compounds but, as mentioned above, it is to be understood that the teachings of the present disclosure are capable of use in various other combinations, modifications, and environments and is capable of changes or modifications within the scope of the inventive concept as expressed herein, commensurate with the above teachings and/or the skill or knowledge of the relevant art. The embodiments described hereinabove are further intended to explain best modes known of practicing the invention and to enable others skilled in the art to utilize the invention in such, or other, embodiments and with the various modifications required by the particular applications or uses of the invention. Accordingly, the description is not intended to limit the invention to the form disclosed herein. All references cited herein are incorporated by reference as if fully set forth in this disclosure.

### REFERENCES

1. Reaven, G. M. (1988). Role of insulin resistance in human disease. Diabetes 37, 1595-1606.
2. Reaven, G. M. (1991). Insulin resistance, hyperinsulinemia, hypertriglyceridemia, and hypertension. Diabetes Care 14, 195-202.
3. DeFronzo, R. A., and Ferrannini, E. (1991). Insulin resistance: A multifaceted syndrome responsible for NIDDM, obesity, hypertension, dyslipidemia, and atherosclerotic cardiovascular disease. Diabetes Care 14, 173-194.
4. Garvey WT, Kwon S, Zheng D, Shaughnessy S, Wallace P, Pugh K, Jenkins AJ, Klein RL, Liao Y. (2003). The Effects of Insulin Resistance and Type 2 Diabetes Mellitus on Lipoprotein Subclass Particle Size and Concentration Determined by Nuclear Magnetic Resonance. Diabetes, 52, 453-462.
5. Hunter, S.J., and Garvey, W.T. (1998). Insulin action and insulin resistance: diseases involving defects in insulin receptors, signal transduction, and the glucose transport effector system. American Journal of Medicine. 105:331-345*.*
6. O'Brien, R. M., and Granner, D. K. (1996). Regulation of gene expression by insulin. Physiol. Rev. 76, 1109-1161.
7. Laville, M., Auboeuf, D., Khalfallah, Y., Vega, N., Rou, J. P., and Vidal, H. (1996). Acute regulation by insulin of phosphatidylinositol-3-kinase, Rad, Glut 4, and lipoprotein lipase mRNA levels in human muscle. J. Clin. Invest. 98,43-49.
8. Bao, S., Kennedy, A., Wojciechowski, B., Wallace, P., Ganaway, E., and Garvey, W. T. (1998). Expression of mRNAs encoding uncoupling proteins in human skeletal muscle: effects of obesity and diabetes. Diabetes 47, 1935-1940.
9. Ohkura, T., Hijikuro, M., Yamanoto, A., and Miki, K. (1994). Molecular cloning of a novel thyroid/steroid receptor superfamily gene from cultured rat neuronal cells. Biochem. Biophys. Res. Commun. 205, 1959-1965.
10. Wilson, T. E., Fahrner, T. J., Johnston, M., and Milbrandt, J. (1991). Identification of the DNA binding site for NGFI-B by genetic selection in Yeast. Science 252, 1296-1300.
11. Mangelsdorf, D. J., and Evans, R. M. (1995). The RXR heterodimers and orphan receptors. Cell 83, 841-850.
12. Perlmann, T., and Jansson, L. (1995). A novel pathway for vitamin A signaling mediated by RXR heterodimerization with NGFI-B and NURR1. Genes Dev. 9, 769-782.
13. Philips, A., Lesage, S., Gingras, R., Maira, M.-H., Gauthier, Y., Hugo, P., and Drouin, J. (1997). Novel dimeric Nur77 signaling mechanism in endocrine and lymphoid cells. Mol. Cell. Biol. 17, 5946-5951.
14. Milbrandt J. (1988). Nerve growth factor induces a gene homologous to the glucocorticoid receptor gene. Neuron 1, 183-188.
15. Hazel T. G., Nathans D., Lau L. F. (1988). A gene inducible by serum growth factors encodes a member of the steroid and thyroid hormone receptor superfamily. Proc. Natl. Acad. Sci. USA 85, 8444-8448.
16. Scearce L. M., Laz T. M., Hazel T. G., Lau L. F., Taub R. (1993). RNR-1, a nuclear receptor in the NGFI-N/Nur77 family that is rapidly induced in regenerating liver. J. Biol. Chem. 268, 8855-8861.
17. Hedvat C. V., Irving S. G. (1995). The isolation and characterization of MINOR, a novel mitogen-induced nuclear orphan receptor. Mol. Endocrinol. 9, 1692-1700.
18. Tofovic S. P., and Jackson, E. K. (2003). Rat models of the metabolic syndrome. Methods in Molecular Medicine. 86, 29-46**.**
19. Tschop, M., and Heiman, M. L. (2001). Rodent obesity models: an overview. Experimental & Clinical Endocrinology & Diabetes. 109, 307-319.
20. Moore, G. B., Chapman, H., Holder, J. C., Lister, C. A., Piercy, V., Smith, S. A., and Clapham, J. C. (2001). Differential regulation of adipocytokine mRNAs by rosiglitazone in db/db mice. Biochemical & Biophysical Research Communications. 286, 735-741.
21. Hunter, S., and Garvey, W. T. (1998). Insulin action and insulin resistance: Diseases involving defects in insulin receptors, signal transduction, and the glucose transport effector system. Am. J. Med. 105, 331-345.
22. Saltiel, A. R., and Olefsky, J. M. (1996). Thiazolidinediones in the treatment of insulin resistance and type II diabetes. Diabetes 45, 1661-1669.
23. Lehmann, J. M., Moore, L. B., Smith-Oliver, T. A., Wilkinson, W.O., Wilson, T. M., and Kliewer, S. A. (1995). An antidiabetic thazolidinedione is a high affinity ligand for PPARγ. J. Biol. Chem. 270, 12953-12956.
24. Braissant, O., Foufelle, F., Scotto, C., Dauca, M., and Wahli, W. (1996). Differential expression of peroxisome proliferators-activated receptors (PPARs): tissue distribution of PPAR-alpha, -beta and -gamma in the adult rat. Endocrinology 137, 354-366.
25. Mayor, P., Maianu, L., and Garvey, W. T. 1992. Glucose and insulin chronically regulate insulin action via different mechanisms in BC3H1 myocytes: Effects on glucose transporter gene expression. Diabetes 41: 274-285.
26. Nelson, B. A., Robinson, K. A., and Buse, M. G. 2000. High glucose and glucosamine induce insulin resistance via different mechanisms in 3T3-L1 adipocytes. Diabetes 49: 981-991.

**TABLE 1: Microarray Results Comparing Gene Expression in Skeletal Muscle Among Insulin Sensitive, Insulin Resistant, and Type 2 Diabetic Humans: p values for statistically significant differential expression**

| | **Basal vs Insulin Stimulation** | | | Basal Tissues | | Insulin-Stimulated Tissues | |
|---|---|---|---|---|---|---|---|
| | IS | IR | DM | **IS vs IR** | **IS vs DB** | **IS vs IR** | **IS vs DB** |
| MINOR | .0001 | .0548 | .0020 | .0053 | .0033 | .0023 | .0053 |
| TR3 | <.0001 | <.0001 | .0002 | NS | NS | .0105 | .0375 |

| | | | | | | | |
|---|---|---|---|---|---|---|---|
| Note: IS = insulin sensitive as assessed by clamp study and normoglycemic; IR = insulin resistant as assessed by clamp study and normoglycemic; DB = insulin-resistant patients with untreated Type 2 Diabetes. NS = not significant. | | | | | | | |
| Bold type indicates conditions being compared. | | | | | | | |

## Claims

1. Use of a compound for the manufacture of a medicament for treating or preventing insulin resistance in a subject, the compound being capable of activating an insulin responsive gene selected from the group consisting of MINOR, TR3 and MINOR and TR3.

2. The use of claim 1 where the compound is at least a fragment of said insulin responsive gene and the medicament is introduced into a tissue of the subject.

3. The use of claim 2 where said tissue is skeletal muscle or adipose tissue.

4. The use of any one of the preceding claims wherein the medicament further comprises at least one secondary therapeutic agent.

5. The use of claim 4 where said secondary therapeutic agent is selected from the group consisting of metformin, sulforylurea and insulin.

6. Use of a compound for the manufacture of a medicament for treating or preventing a disease state associated with insulin resistance in a subject, the compound being capable of activating an insulin responsive gene selected from the group consisting of MINOR, TR3 and MINOR and TR3.

7. The use of claim 6 where the compound is at least a fragment of said insulin responsive gene and the medicament is introduced into a tissue of said subject.

8. The use of claim 7 where said tissue is skeletal muscle or adipose tissue.

9. The use of any one of claims 6 to 8 wherein the medicament further comprises at least one secondary therapeutic agent.

10. The use of claim 9 where said secondary therapeutic agent is selected from the group consisting of metformin, sulforylurea and insulin.

11. The use of any one of claims 6 to 10 where said disease state associated with insulin resistance is selected from the group consisting of: metabolic syndrome, pre-diabetes, polycystic ovary syndrome, type 2 diabetes, dyslipidemia, obesity, infertility, inflammatory disorders, cancer, inflammatory diseases, Alzheimer's disease, hypertension, atherosclerosis, cardiovascular disease and peripheral vascular disease.

12. A method for identifying a compound which activates an insulin responsive gene, said method comprising:
a. providing a cell expressing at least a portion of said insulin responsive gene, said insulin responsive gene selected from the group consisting of: MINOR, TR3 and MINOR and TR3;
b. incubating said cell with said compound and;
c. measuring a response to said compound.

13. The method of claim 12 where said response is an increase in the expression of at least one of MINOR or TR3.

14. The method of claim 14 where said response is selected from the group consisting of: an increase in the expression of a polypeptide encoded by said insulin responsive gene, an increase in the activity of a polypeptide encoded by said insulin responsive gene, a functional response mediated by said insulin responsive gene.

15. The method of claim 14 where said functional response is at least one of the responses selected from the group consisting of: increased glucose uptake, increased expression of a transporter involved in glucose regulation, increased translocation of a transporter involved in glucose regulation to the cell membrane and increased activity of downstream signal transduction pathways regulated by said insulin responsive gene.

16. The method of claim 15 where said transporter is a GLUT4 transporter.

17. A method for identifying a compound useful for the treatment or prevention of insulin resistance, said method comprising the step of determining whether the compound interacts with the polypeptide product of a gene selected from the group consisting of: MINOR, TR3 and MINOR and TR3.

18. The method of claim 17 where said interaction is a direct interaction or an indirect interaction.

19. A method for identifying a compound useful for the treatment or prevention of insulin resistance, said method comprising the step of determining whether the compound activates a gene selected from the group consisting of: MINOR, TR3 and MINOR and TR3.

20. A method for identifying a compound useful for the treatment or prevention of a disease state associated with insulin resistance, said method comprising the step of determining whether the compound interacts with the polypeptide product of a gene selected from the group consisting of: MINOR, TR3 and MINOR and TR3.

21. The method of claim 20 where said interaction is a direct interaction or an indirect interaction.

22. A method for identifying a compound useful for the treatment or prevention of a disease state associated with insulin resistance, said method comprising the step of determining whether the compound activates a gene selected from the group consisting of: MINOR, TR3 and MINOR and TR3.

23. The method of any one of claims 20 to 22 where said disease state associated with insulin resistance is selected from the group consisting of: metabolic syndrome, pre-diabetes, polycystic ovary syndrome, type 2 diabetes, dyslipidemia, obesity, infertility, inflammatory disorders, cancer, inflammatory diseases, Alzheimer's disease, hypertension, atherosclerosis, cardiovascular disease and peripheral vascular disease.

24. Use of a compound which was identified by the method of any one of claims 17 to 19 for treating or preventing insulin resistance.

25. Use of a compound which was identified by the method of any one of claims 20 to 23 for treating or preventing a disease state associated with insulin resistance.

26. The use of claim 25 where said disease state associated with insulin resistance is selected from the group consisting of: metabolic syndrome, pre-diabetes, polycystic ovary syndrome, type 2 diabetes, dyslipidemia, obesity, infertility, inflammatory disorders, cancer, inflammatory diseases, Alzheimer's disease, hypertension, atherosclerosis, cardiovascular disease and peripheral vascular disease.


SEQUENCE LISTING

<110> UAB Research Foundation
F. Hoffmann-La Roche Ltd.

<120> Methods for the Treatment of Insulin Resistance and Disease States
Characterized by Insulin Resistance

<130> WA/48173

<150> US 60/664,792
<151> 2005-03-24

<160> 8

<170> PatentIn version 3.1

<210> 1
<211> 4977
<212> DNA
<213> Homo sapiens

<220>
<221> 5'UTR
<222> (1)..(209)
<223>

<220>
<221> CDS
<222> (210)..(1973)
<223>

<220>
<221> 3'UTR
<222> (1974)..(4977)
<223>

<220>
<221> polyA_signal
<222> (4953)..(4958)
<223>

<220>
<221> polyA_site
<222> (4977)..(4977)
<223>

<400> 1
caagataccc tccaggccct catcaccttt tttcaagtca agatttcatc ccatacatgc 60

atgactcaat cagatttgga aatgtggata tgccctgtcg tccaagccca atatagccct 120

tcccctccag gttccagtta tgcggtcgtc agacatacag ctcggaatac accacggaga 180

tcatgaaccc cgactacacc aagctgacc atg gac ctt ggc agc act gag atc 233
Met Asp Leu Gly Ser Thr Glu Ile
1               5

acg gct aca gcc acc acg tcc ctg ccc agc atc agt acc ttt gtg gag 281
Thr Ala Thr Ala Thr Thr Ser Leu Pro Ser Ile Ser Thr Phe Val Glu
    10                  15                  20

ggc tac tcg agc aac tac gaa ctc aag cct tcc tgc gtg tac caa atg 329
Gly Tyr Ser Ser Asn Tyr Glu Leu Lys Pro Ser Cys Val Tyr Gln Met
25                  30                  35                  40

cag cgg ccc ttg atc aaa gtg gag gag ggg cgg gcg ccc agc tac cat 377
Gln Arg Pro Leu Ile Lys Val Glu Glu Gly Arg Ala Pro Ser Tyr His
                45                  50                  55

cac cat cac cac cac cac cac cac cac cac cac cat cac cag cag cag 425
His His His His His His His His His His His His His Gln Gln Gln
            60                  65                  70

cat cag cag cca tcc att cct cca gcc tcc agc ccg gag gac gag gtg 473
His Gln Gln Pro Ser Ile Pro Pro Ala Ser Ser Pro Glu Asp Glu Val
        75                  80                  85

ctg ccc agc acc tcc atg tac ttc aag cag tcc cca ccg tcc acc ccc 521
Leu Pro Ser Thr Ser Met Tyr Phe Lys Gln Ser Pro Pro Ser Thr Pro
    90                  95                  100

acc acg ccg gcc ttc ccc ccg cag gcg ggg gcg tta tgg gac gag gca 569
Thr Thr Pro Ala Phe Pro Pro Gln Ala Gly Ala Leu Trp Asp Glu Ala
105                 110                 115                 120

ctg ccc tcg gcg ccc ggc tgc atc gca ccc ggc ccg ctg ctg gac ccg 617
Leu Pro Ser Ala Pro Gly Cys Ile Ala Pro Gly Pro Leu Leu Asp Pro
                125                 130                 135

ccg atg aag gcg gtc ccc acg gtg gcc ggc gcg cgc ttc ccg ctc ttc 665
Pro Met Lys Ala Val Pro Thr Val Ala Gly Ala Arg Phe Pro Leu Phe
            140                 145                 150

cac ttc aag ccc tcg ccg ccg cat ccc ccc gcg ccc agc ccg gcc ggc 713
His Phe Lys Pro Ser Pro Pro His Pro Pro Ala Pro Ser Pro Ala Gly
        155                 160                 165

ggc cac cac ctc ggc tac gac ccg acg gcc gct gcc gcg ctc agc ctg 761
Gly His His Leu Gly Tyr Asp Pro Thr Ala Ala Ala Ala Leu Ser Leu
    170                 175                 180

ccg ctg gga gcc gca gcc gcc gcg ggc agc cag gcc gcc gcg ctt gag 809
Pro Leu Gly Ala Ala Ala Ala Ala Gly Ser Gln Ala Ala Ala Leu Glu
185                 190                 195                 200

ggc cac ccg tac ggg ctg ccg ctg gcc aag agg gcg gcc ccg ctg gcc 857
Gly His Pro Tyr Gly Leu Pro Leu Ala Lys Arg Ala Ala Pro Leu Ala
                205                 210                 215

ttc ccg cct ctc ggc ctc acg ccc tcc cct acc gcg tcc agc ctg ctg 905
Phe Pro Pro Leu Gly Leu Thr Pro Ser Pro Thr Ala Ser Ser Leu Leu
            220                 225                 230

ggc gag agt ccc agc ctg ccg tcg ccg ccc agc agg agc tcg tcg tct 953
Gly Glu Ser Pro Ser Leu Pro Ser Pro Pro Ser Arg Ser Ser Ser Ser
        235                 240                 245

ggc gag ggc acg tgt gcc gtg tgc ggg gac aac gcc gcc tgc cag cac 1001
Gly Glu Gly Thr Cys Ala Val Cys Gly Asp Asn Ala Ala Cys Gln His
    250                 255                 260

tac ggc gtg cga acc tgc gag ggc tgc aag ggc ttt ttc aag aga aca 1049
Tyr Gly Val Arg Thr Cys Glu Gly Cys Lys Gly Phe Phe Lys Arg Thr
265                 270                 275                 280

gtg cag aaa aat gca aaa tat gtt tgc ctg gca aat aaa aac tgc cca 1097
Val Gln Lys Asn Ala Lys Tyr Val Cys Leu Ala Asn Lys Asn Cys Pro
                285                 290                 295

gta gac aag aga cgt cga aac cga tgt cag tac tgt cga ttt cag aag 1145
Val Asp Lys Arg Arg Arg Asn Arg Cys Gln Tyr Cys Arg Phe Gln Lys
            300                 305                 310

tgt ctc agt gtt gga atg gta aaa gaa gtt gtc cgt aca gat agt ctg 1193
Cys Leu Ser Val Gly Met Val Lys Glu Val Val Arg Thr Asp Ser Leu
        315                 320                 325

aaa ggg agg aga ggt cgt ctg cct tcc aaa cca aag agc cca tta caa 1241
Lys Gly Arg Arg Gly Arg Leu Pro Ser Lys Pro Lys Ser Pro Leu Gln
    330                 335                 340

cag gaa cct tct cag ccc tct cca cct tct cct cca atc tgc atg atg 1289
Gln Glu Pro Ser Gln Pro Ser Pro Pro Ser Pro Pro Ile Cys Met Met
345                 350                 355                 360

aat gct ctt gtc cga gct tta aca gac tca aca ccc aga gat ctt gat 1337
Asn Ala Leu Val Arg Ala Leu Thr Asp Ser Thr Pro Arg Asp Leu Asp
                365                 370                 375

tat tcc aga tac tgt ccc act gac cag gct gct gca ggc aca gat gct 1385
Tyr Ser Arg Tyr Cys Pro Thr Asp Gln Ala Ala Ala Gly Thr Asp Ala
            380                 385                 390

gag cat gtg caa caa ttc tac aac ctc ctg aca gcc tcc att gat gta 1433
Glu His Val Gln Gln Phe Tyr Asn Leu Leu Thr Ala Ser Ile Asp Val
        395                 400                 405

tcc aga agc tgg gca gaa aag att ccg gga ttt act gat ctc ccc aaa 1481
Ser Arg Ser Trp Ala Glu Lys Ile Pro Gly Phe Thr Asp Leu Pro Lys
    410                 415                 420

gaa gat cag aca tta ctt att gaa tca gcc ttt ttg gag ctg ttt gtc 1529
Glu Asp Gln Thr Leu Leu Ile Glu Ser Ala Phe Leu Glu Leu Phe Val
425                 430                 435                 440

ctc aga ctt tcc atc agg tca aac act gct gaa gat aag ttt gtg ttc 1577
Leu Arg Leu Ser Ile Arg Ser Asn Thr Ala Glu Asp Lys Phe Val Phe
                445                 450                 455

tgc aat gga ctt gtc ctg cat cga ctt cag tgc ctt cgt gga ttt ggg 1625
Cys Asn Gly Leu Val Leu His Arg Leu Gln Cys Leu Arg Gly Phe Gly
            460                 465                 470

gag tgg ctc gac tct att aaa gac ttt tcc tta aat ttg cag agc ctg 1673
Glu Trp Leu Asp Ser Ile Lys Asp Phe Ser Leu Asn Leu Gln Ser Leu
        475                 480                 485

aac ctt gat atc caa gcc tta gcc tgc ctg tca gca ctg agc atg atc 1721
Asn Leu Asp Ile Gln Ala Leu Ala Cys Leu Ser Ala Leu Ser Met Ile
    490                 495                 500

aca gaa aga cat ggg tta aaa gaa cca aag aga gtc gaa gag cta tgc 1769
Thr Glu Arg His Gly Leu Lys Glu Pro Lys Arg Val Glu Glu Leu Cys
505                 510                 515                 520

aac aag atc aca agc agt tta aaa gac cac cag agt aag gga cag gct 1817
Asn Lys Ile Thr Ser Ser Leu Lys Asp His Gln Ser Lys Gly Gln Ala
                525                 530                 535

ctg gag ccc acc gag tcc aag gtc ctg ggt gcc ctg gta gaa ctg agg 1865
Leu Glu Pro Thr Glu Ser Lys Val Leu Gly Ala Leu Val Glu Leu Arg
            540                 545                 550

aag atc tgc acc ctg ggc ctc cag cgc atc ttc tac ctg aag ctg gaa 1913
Lys Ile Cys Thr Leu Gly Leu Gln Arg Ile Phe Tyr Leu Lys Leu Glu
        555                 560                 565

gac ttg gtg tct cca cct tcc atc att gac aag ctc ttc ctg gac acc 1961
Asp Leu Val Ser Pro Pro Ser Ile Ile Asp Lys Leu Phe Leu Asp Thr
    570                 575                 580

cta cct ttc taa tcaggagcag tggagcagtg agctgcctcc tctcctagca 2013
Leu Pro Phe
585

cctgcttgct acgcagcaaa gggataggtt tggaaaccta tcatttcctg tccttcctta 2073

agaggaaaag cagctcctgt agaaagcaaa gactttcttt tttttctggc tcttttcctt 2133

acaacctaaa gccagaaaac ttgcagagta ttgtgttggg gttgtgtttt atatttaggc 2193

attgggggat ggggtgggag ggggttatag ttcatgaggg ttttctaaga aattgctaac 2253

aaagcacttt tggacaatgc tatcccagca ggaaaaaaaa ggataatata actgttttaa 2313

aactctttct ggggaatcca attatagttg ctttgtattt aaaaacaaga acagccaagg 2373

gttgttcgcc agggtaggat gtgtcttaaa gattggtccc ttgaaaatat gcttcctgta 2433

tcaaaggtac gtatgtggtg caaacaaggc agaaacttcc ttttaatttc cttcttcctt 2493

tattttaaca aatggtgaaa gatggaggat tacctacaat cagacatggc aaaacaatat 2553

ggctgtttgc ttcataacag ctgcaatttt taaagtgctg tcttactaag tcttgttata 2613

ctctcttatc tatatgccgg aaataaaaag gaggcagtca tgttagcaaa tgacacgtta 2673

atatccctag cagaggctgt gttcaccttc cctgtcgatc ccttctgagg tatggcccat 2733

ccaagacttt taggccattc ttgatggaac cagatccctg ccctgactgt ccagctatcc 2793

tgaaagtgga tcagattata aactggatta catgtaactg ttttggttgt gttctatcaa 2853

ccccaccaga gttccctaaa cttgcttcag ttatagtaac tgactggtat atcattcaga 2913

agcgccataa gtcagttgag tattgatcct agataagaac atgcaatcag aggactggtc 2973

atacagggta agcaccaggg acaataagga tttttataga tataatttaa tttttgttat 3033

tggttaagga gacaattttg gagagcaagc aaatcttctt tttaaaaaat agtatgaatg 3093

tgaatactag aaaagattta agaaatagta tgagtgtgag tcataggaag gattagtggg 3153

ctgcgtttca acattccgtg ttcgtactcc cttttgtatg tttctactgt taatgccata 3213

ttactatgag ataatttgtt gcatagtgtc cttatttgta taaacatttg tatgcacgtt 3273

atattgtaat agctttgcct gtatttattg caagaccacc agctcctgga agctgagtta 3333

cagagtaatt aaatggggtg ttcacagtga cttggataca ccaattagaa attaaataag 3393

caaatatata tatatatata taaatatagc aggttacata tatatattta taatgtgtct 3453

ttttattaac catttgtaca ataaatgtca cttcccatgc cgttatttta tggttcattt 3513

gcagtgactt ttaaggcagt actgtttagc actttgatat taaaattttg cttatgtttt 3573

gctaaattcg aataatgttt gaagattttt aggtctaaaa gtctttatat tatatacgct 3633

gtatcaagtc aaaatatctt tggccatttt gctaagaaac aaactttgaa tgtcaaactg 3693

atgtcacagt agtttttgtt agctttaaat catttttgct ttagtctttt taaaggaaaa 3753

taacaaaact atgctgttta tattgcatta attatacaat caaacaaatg ccaaatgaat 3813

tgcctaattg cgcaagataa cccagatagg aaatcatatg tttttttcca agagtcattc 3873

taatatttga ttatgttatg tgtgctttta tgaaagattg ttatttttat atatcaagat 3933

gatagaacct ggaatgttag gattttgaaa tgttagactt ggaaggggcc tggtctgtca 3993

actagtccaa ccccttaaaa ttcatagagg acgaaactgg ggcccaattg aagggtgaag 4053

agttactcaa ggtcaaacag ctggtaacag aatcaagact aagacctaat ttacctttcc 4113

atactctttt tttttctcaa cttcatctat ataaaatcag gcttttaaac ataaccacta 4173

atatttacct gaagataacc atgagtaaag tatacttttg cattaatttt ttgagtatat 4233

gcaaacataa taaatattat taaatatcaa ggaaagctaa catttcatac aagatagctt 4293

cagaccaaat tcaaattgaa tttgaataaa ttagaaatac tgtgcataca taaccttctt 4353

gtgcaccatg agtatttgga aagttaatcc ttgtttttgt cgtgtctata gagaacaaac 4413

aaataaaaac agagccctag agaaatggcg ttacttttta tttttacacc catcagattt 4473

aaggaaaaga ctttttagcc attataatct agtggttgga aggaatgaag aagctttttt 4533

agtaataggt ccagatatga gtgctaaaaa taaagatgat agcatgttct tctgtcttcc 4593

atagttatta caactatgag agcctcccaa gtcatcttat caactcaacc cccttttttt 4653

tgtcttaatg ttgcacataa gtttatacag agtggatgac cacactagca cagaagagaa 4713

caacatgtat taaagcaggt gattcctccc ttggcgggag agctctctca gtgtgaacat 4773

gccttctgtg ggcggaaatc aggaagccac cagctgttaa tggagagtgc cttgctttta 4833

tttcagacag cagagttttc caaagtttct ctgctcctct aacagcattg ctctttagtg 4893

tgtgttaacc tgtggtttga aagaaatgct cttgtacatt aacaatgtaa atttaaatga 4953

ttaaattaca ttttatcaat ggca 4977

<210> 2
<211> 587
<212> PRT
<213> Homo sapiens

<400> 2

Met Asp Leu Gly Ser Thr Glu Ile Thr Ala Thr Ala Thr Thr Ser Leu
1               5                   10                  15

Pro Ser Ile Ser Thr Phe Val Glu Gly Tyr Ser Ser Asn Tyr Glu Leu
            20                  25                  30

Lys Pro Ser Cys Val Tyr Gln Met Gln Arg Pro Leu Ile Lys Val Glu
        35                  40                  45

Glu Gly Arg Ala Pro Ser Tyr His His His His His His His His His
    50                  55                  60

His His His His His Gln Gln Gln His Gln Gln Pro Ser Ile Pro Pro
65                  70                  75                  80

Ala Ser Ser Pro Glu Asp Glu Val Leu Pro Ser Thr Ser Met Tyr Phe
                85                  90                  95

Lys Gln Ser Pro Pro Ser Thr Pro Thr Thr Pro Ala Phe Pro Pro Gln
            100                 105                 110

Ala Gly Ala Leu Trp Asp Glu Ala Leu Pro Ser Ala Pro Gly Cys Ile
        115                 120                 125

Ala Pro Gly Pro Leu Leu Asp Pro Pro Met Lys Ala Val Pro Thr Val
    130                 135                 140

Ala Gly Ala Arg Phe Pro Leu Phe His Phe Lys Pro Ser Pro Pro His
145                 150                 155                 160

Pro Pro Ala Pro Ser Pro Ala Gly Gly His His Leu Gly Tyr Asp Pro
                165                 170                 175

Thr Ala Ala Ala Ala Leu Ser Leu Pro Leu Gly Ala Ala Ala Ala Ala
            180                 185                 190

Gly Ser Gln Ala Ala Ala Leu Glu Gly His Pro Tyr Gly Leu Pro Leu
        195                 200                 205

Ala Lys Arg Ala Ala Pro Leu Ala Phe Pro Pro Leu Gly Leu Thr Pro
    210                 215                 220

Ser Pro Thr Ala Ser Ser Leu Leu Gly Glu Ser Pro Ser Leu Pro Ser
225                 230                 235                 240

Pro Pro Ser Arg Ser Ser Ser Ser Gly Glu Gly Thr Cys Ala Val Cys
                245                 250                 255

Gly Asp Asn Ala Ala Cys Gln His Tyr Gly Val Arg Thr Cys Glu Gly
            260                 265                 270

Cys Lys Gly Phe Phe Lys Arg Thr Val Gln Lys Asn Ala Lys Tyr Val
        275                 280                 285

Cys Leu Ala Asn Lys Asn Cys Pro Val Asp Lys Arg Arg Arg Asn Arg
    290                 295                 300

Cys Gln Tyr Cys Arg Phe Gln Lys Cys Leu Ser Val Gly Met Val Lys
305                 310                 315                 320

Glu Val Val Arg Thr Asp Ser Leu Lys Gly Arg Arg Gly Arg Leu Pro
                325                 330                 335

Ser Lys Pro Lys Ser Pro Leu Gln Gln Glu Pro Ser Gln Pro Ser Pro
            340                 345                 350

Pro Ser Pro Pro Ile Cys Met Met Asn Ala Leu Val Arg Ala Leu Thr
        355                 360                 365

Asp Ser Thr Pro Arg Asp Leu Asp Tyr Ser Arg Tyr Cys Pro Thr Asp
    370                 375                 380

Gln Ala Ala Ala Gly Thr Asp Ala Glu His Val Gln Gln Phe Tyr Asn
385                 390                 395                 400

Leu Leu Thr Ala Ser Ile Asp Val Ser Arg Ser Trp Ala Glu Lys Ile
                405                 410                 415

Pro Gly Phe Thr Asp Leu Pro Lys Glu Asp Gln Thr Leu Leu Ile Glu
            420                 425                 430

Ser Ala Phe Leu Glu Leu Phe Val Leu Arg Leu Ser Ile Arg Ser Asn
        435                 440                 445

Thr Ala Glu Asp Lys Phe Val Phe Cys Asn Gly Leu Val Leu His Arg
    450                 455                 460

Leu Gln Cys Leu Arg Gly Phe Gly Glu Trp Leu Asp Ser Ile Lys Asp
465                 470                 475                 480

Phe Ser Leu Asn Leu Gln Ser Leu Asn Leu Asp Ile Gln Ala Leu Ala
                485                 490                 495

Cys Leu Ser Ala Leu Ser Met Ile Thr Glu Arg His Gly Leu Lys Glu
            500                 505                 510

Pro Lys Arg Val Glu Glu Leu Cys Asn Lys Ile Thr Ser Ser Leu Lys
        515                 520                 525

Asp His Gln Ser Lys Gly Gln Ala Leu Glu Pro Thr Glu Ser Lys Val
    530                 535                 540

Leu Gly Ala Leu Val Glu Leu Arg Lys Ile Cys Thr Leu Gly Leu Gln

545                 550                 555                 560

Arg Ile Phe Tyr Leu Lys Leu Glu Asp Leu Val Ser Pro Pro Ser Ile
                565                 570                 575

Ile Asp Lys Leu Phe Leu Asp Thr Leu Pro Phe
            580                 585

<210>  3
<211>  2464
<212>  DNA
<213>  Homo sapiens

<220>
<221>  CDS
<222>  (111)..(1907)
<223>

<220>
<221>  polyA_signal
<222>  (2442)..(2447)
<223>

<220>
<221>  polyA_site
<222>  (2464)..(2464)
<223>

<400>  3
cggaaacttg ggggagtgca cagaagaact tcgggagcgc acgcgggacc agggaccagg      60

ctgagactcg gggcgccagt ccgggcaggg gcagcgggac gcggccggag atg ccc      116
                                                       Met Pro
                                                       1

tgt atc caa gcc caa tat ggg aca cca gca ccg agt ccg gga ccc cgt      164
Cys Ile Gln Ala Gln Tyr Gly Thr Pro Ala Pro Ser Pro Gly Pro Arg
        5                   10                  15

gac cac ctg gca agc gac ccc ctg acc cct gag ttc atc aag ccc acc      212
Asp His Leu Ala Ser Asp Pro Leu Thr Pro Glu Phe Ile Lys Pro Thr
    20                  25                  30

atg gac ctg gcc agc ccc gag gca gcc ccc gct gcc ccc act gcc ctg      260
Met Asp Leu Ala Ser Pro Glu Ala Ala Pro Ala Ala Pro Thr Ala Leu
35                  40                  45                  50

ccc agc ttc agc acg ttc atg gac ggc tac aca gga gag ttt gac acc      308
Pro Ser Phe Ser Thr Phe Met Asp Gly Tyr Thr Gly Glu Phe Asp Thr
                55                  60                  65

ttc ctc tac cag ctg cca gga aca gtc cag cca tgc tcc tca gcc tcc 356
Phe Leu Tyr Gln Leu Pro Gly Thr Val Gln Pro Cys Ser Ser Ala Ser
            70                  75                  80

tcc tcg gcc tcc tcc aca tcc tcg tcc tca gcc acc tcc cct gcc tct 404
Ser Ser Ala Ser Ser Thr Ser Ser Ser Ser Ala Thr Ser Pro Ala Ser
        85                  90                  95

gcc tcc ttc aag ttc gag gac ttc cag gtg tac ggc tgc tac ccc ggc 452
Ala Ser Phe Lys Phe Glu Asp Phe Gln Val Tyr Gly Cys Tyr Pro Gly
    100                 105                 110

ccc ctg agc ggc cca gtg gat gag gcc ctg tcc tcc agt ggc tct gac 500
Pro Leu Ser Gly Pro Val Asp Glu Ala Leu Ser Ser Ser Gly Ser Asp
115                 120                 125                 130

tac tat ggc agc ccc tgc tcg gcc ccg tcg ccc tcc acg ccc agc ttc 548
Tyr Tyr Gly Ser Pro Cys Ser Ala Pro Ser Pro Ser Thr Pro Ser Phe
                135                 140                 145

cag ccg ccc cag ctc tct ccc tgg gat ggc tcc ttc ggc cac ttc tcg 596
Gln Pro Pro Gln Leu Ser Pro Trp Asp Gly Ser Phe Gly His Phe Ser
            150                 155                 160

ccc agc cag act tac gaa ggc ctg cgg gca tgg aca gag cag ctg ccc 644
Pro Ser Gln Thr Tyr Glu Gly Leu Arg Ala Trp Thr Glu Gln Leu Pro
        165                 170                 175

aaa gcc tct ggg ccc cca cag cct cca gcc ttc ttt tcc ttc agt cct 692
Lys Ala Ser Gly Pro Pro Gln Pro Pro Ala Phe Phe Ser Phe Ser Pro
    180                 185                 190

ccc act ggc ccc agc ccc agc ctg gcc cag agc ccc ctg aag ttg ttc 740
Pro Thr Gly Pro Ser Pro Ser Leu Ala Gln Ser Pro Leu Lys Leu Phe
195                 200                 205                 210

ccc tca cag gcc acc cac cag ctg ggg gag gga gag agc tat tcc atg 788
Pro Ser Gln Ala Thr His Gln Leu Gly Glu Gly Glu Ser Tyr Ser Met
                215                 220                 225

cct acg gcc ttc cca ggt ttg gca ccc act tct cca cac ctt gag ggc 836
Pro Thr Ala Phe Pro Gly Leu Ala Pro Thr Ser Pro His Leu Glu Gly
            230                 235                 240

tcg ggg ata ctg gat aca ccc gtg acc tca acc aag gcc cgg agc ggg 884
Ser Gly Ile Leu Asp Thr Pro Val Thr Ser Thr Lys Ala Arg Ser Gly
        245                 250                 255

gcc cca ggt cca agt gaa ggc cgc tgt gct gtg tgt ggg gac aac gct 932
Ala Pro Gly Pro Ser Glu Gly Arg Cys Ala Val Cys Gly Asp Asn Ala
    260                 265                 270

tca tgc cag cat tat ggt gtc cgc aca tgt gag ggc tgc aag ggc ttc 980
Ser Cys Gln His Tyr Gly Val Arg Thr Cys Glu Gly Cys Lys Gly Phe
275                 280                 285                 290

ttc aag cgc aca gtg cag aaa aac gcc aag tac atc tgc ctg gct aac 1028

Phe Lys Arg Thr Val Gln Lys Asn Ala Lys Tyr Ile Cys Leu Ala Asn
                295                 300                 305

aag gac tgc cct gtg gac aag agg cgg cga aac cgc tgc cag ttc tgc      1076
Lys Asp Cys Pro Val Asp Lys Arg Arg Arg Asn Arg Cys Gln Phe Cys
            310                 315                 320

cgc ttc cag aag tgc ctg gcg gtg ggc atg gtg aag gaa gtt gtc cga      1124
Arg Phe Gln Lys Cys Leu Ala Val Gly Met Val Lys Glu Val Val Arg
        325                 330                 335

aca gac agc ctg aag ggg cgg cgg ggc cgg cta cct tca aaa ccc aag      1172
Thr Asp Ser Leu Lys Gly Arg Arg Gly Arg Leu Pro Ser Lys Pro Lys
    340                 345                 350

cag ccc cca gat gcc tcc cct gcc aat ctc ctc act tcc ctg gtc ctt      1220
Gln Pro Pro Asp Ala Ser Pro Ala Asn Leu Leu Thr Ser Leu Val Leu
355                 360                 365                 370

gca cac ctg gat tca ggg ccc agc act gcc aaa ctg gac tac tcc aag      1268
Ala His Leu Asp Ser Gly Pro Ser Thr Ala Lys Leu Asp Tyr Ser Lys
                375                 380                 385

ttc cag gag ctg gtg ctg ccc cac ttt ggg aag gaa gat gct ggg gat      1316
Phe Gln Glu Leu Val Leu Pro His Phe Gly Lys Glu Asp Ala Gly Asp
            390                 395                 400

gta cag cag ttc tac gac ctg ctc tcc ggt tct ctg gag gtc atc cga      1364
Val Gln Gln Phe Tyr Asp Leu Leu Ser Gly Ser Leu Glu Val Ile Arg
        405                 410                 415

aag tgg gcg gag aag atc cct ggc ttt gct gag ctg tca ccg gct gac      1412
Lys Trp Ala Glu Lys Ile Pro Gly Phe Ala Glu Leu Ser Pro Ala Asp
    420                 425                 430

cag gac ctg ttg ctg gag tcg gcc ttc ctg gag ctc ttc atc ctc cgc      1460
Gln Asp Leu Leu Leu Glu Ser Ala Phe Leu Glu Leu Phe Ile Leu Arg
435                 440                 445                 450

ctg gcg tac agg tct aag cca ggc gag ggc aag ctc atc ttc tgc tca      1508
Leu Ala Tyr Arg Ser Lys Pro Gly Glu Gly Lys Leu Ile Phe Cys Ser
                455                 460                 465

ggc ctg gtg cta cac cgg ctg cag tgt gcc cgt ggc ttc ggg gac tgg      1556
Gly Leu Val Leu His Arg Leu Gln Cys Ala Arg Gly Phe Gly Asp Trp
            470                 475                 480

att gac agt atc ctg gcc ttc tca agg tcc ctg cac agc ttg ctt gtc      1604
Ile Asp Ser Ile Leu Ala Phe Ser Arg Ser Leu His Ser Leu Leu Val
        485                 490                 495

gat gtc cct gcc ttc gcc tgc ctc tct gcc ctt gtc ctc atc acc gac      1652
Asp Val Pro Ala Phe Ala Cys Leu Ser Ala Leu Val Leu Ile Thr Asp
    500                 505                 510

cgg cat ggg ctg cag gag ccg cgg cgg gtg gag gag ctg cag aac cgc      1700
Arg His Gly Leu Gln Glu Pro Arg Arg Val Glu Glu Leu Gln Asn Arg

515                 520                 525                 530

atc gcc agc tgc ctg aag gag cac gtg gca gct gtg gcg ggc gag ccc      1748
Ile Ala Ser Cys Leu Lys Glu His Val Ala Ala Val Ala Gly Glu Pro
                535                 540                 545

cag cca gcc agc tgc ctg tca cgt ctg ttg ggc aaa ctg ccc gag ctg      1796
Gln Pro Ala Ser Cys Leu Ser Arg Leu Leu Gly Lys Leu Pro Glu Leu
            550                 555                 560

cgg acc ctg tgc acc cag ggc ctg cag cgc atc ttc tac ctc aag ctg      1844
Arg Thr Leu Cys Thr Gln Gly Leu Gln Arg Ile Phe Tyr Leu Lys Leu
        565                 570                 575

gag gac ttg gtg ccc cct cca ccc atc att gac aag atc ttc atg gac      1892
Glu Asp Leu Val Pro Pro Pro Pro Ile Ile Asp Lys Ile Phe Met Asp
    580                 585                 590

acg ctg ccc ttc tga cccctgcctg ggaacacgtg tgcacatgcg cactctcata      1947
Thr Leu Pro Phe
595

tgccacccca tgtgccttta gtccacggac ccccagagca cccccaagcc tgggcttgag   2007

ctgcagaatg actccacctt ctcacctgct ccaggaggtt tgcagggagc tcaagccctt   2067

ggggaggggg atgccttcat gggggtgacc ccacgatttg tcttatcccc cccagcctgg   2127

ccccggcctt tatgtttttt gtaagataaa ccgtttttaa cacatagcgc cgtgctgtaa   2187

ataagcccag tgctgctgta aatacaggaa gaaagagctt gaggtgggag cggggctggg   2247

aggaagggat gggccccgcc ttcctgggca gcctttccag cctcctgcct ggctctctct   2307

tcctaccctc cttccacatg tacataaact gtcactctag gaagaagaca aatgacagat   2367

tctgacattt atatttgtgt attttcctgg atttatagta tgtgactttt ctgattaata   2427

tatttaatat attgaataaa aaatagacat gtagttg                            2464

<210>  4
<211>  598
<212>  PRT
<213>  Homo sapiens

<400>  4

Met Pro Cys Ile Gln Ala Gln Tyr Gly Thr Pro Ala Pro Ser Pro Gly
1               5                   10                  15

Pro Arg Asp His Leu Ala Ser Asp Pro Leu Thr Pro Glu Phe Ile Lys
            20                  25                  30

Pro Thr Met Asp Leu Ala Ser Pro Glu Ala Ala Pro Ala Ala Pro Thr

35                  40                  45

Ala Leu Pro Ser Phe Ser Thr Phe Met Asp Gly Tyr Thr Gly Glu Phe
    50                  55                  60

Asp Thr Phe Leu Tyr Gln Leu Pro Gly Thr Val Gln Pro Cys Ser Ser
65                  70                  75                  80

Ala Ser Ser Ser Ala Ser Ser Thr Ser Ser Ser Ser Ala Thr Ser Pro
                85                  90                  95

Ala Ser Ala Ser Phe Lys Phe Glu Asp Phe Gln Val Tyr Gly Cys Tyr
            100                 105                 110

Pro Gly Pro Leu Ser Gly Pro Val Asp Glu Ala Leu Ser Ser Ser Gly
        115                 120                 125

Ser Asp Tyr Tyr Gly Ser Pro Cys Ser Ala Pro Ser Pro Ser Thr Pro
    130                 135                 140

Ser Phe Gln Pro Pro Gln Leu Ser Pro Trp Asp Gly Ser Phe Gly His
145                 150                 155                 160

Phe Ser Pro Ser Gln Thr Tyr Glu Gly Leu Arg Ala Trp Thr Glu Gln
                165                 170                 175

Leu Pro Lys Ala Ser Gly Pro Pro Gln Pro Pro Ala Phe Phe Ser Phe
            180                 185                 190

Ser Pro Pro Thr Gly Pro Ser Pro Ser Leu Ala Gln Ser Pro Leu Lys
        195                 200                 205

Leu Phe Pro Ser Gln Ala Thr His Gln Leu Gly Glu Gly Glu Ser Tyr
    210                 215                 220

Ser Met Pro Thr Ala Phe Pro Gly Leu Ala Pro Thr Ser Pro His Leu
225                 230                 235                 240

Glu Gly Ser Gly Ile Leu Asp Thr Pro Val Thr Ser Thr Lys Ala Arg
                245                 250                 255

Ser Gly Ala Pro Gly Pro Ser Glu Gly Arg Cys Ala Val Cys Gly Asp
            260                 265                 270

Asn Ala Ser Cys Gln His Tyr Gly Val Arg Thr Cys Glu Gly Cys Lys
        275                 280                 285

Gly Phe Phe Lys Arg Thr Val Gln Lys Asn Ala Lys Tyr Ile Cys Leu
    290                 295                 300

Ala Asn Lys Asp Cys Pro Val Asp Lys Arg Arg Arg Asn Arg Cys Gln
305                 310                 315                 320

Phe Cys Arg Phe Gln Lys Cys Leu Ala Val Gly Met Val Lys Glu Val
                325                 330                 335

Val Arg Thr Asp Ser Leu Lys Gly Arg Arg Gly Arg Leu Pro Ser Lys
            340                 345                 350

Pro Lys Gln Pro Pro Asp Ala Ser Pro Ala Asn Leu Leu Thr Ser Leu
        355                 360                 365

Val Leu Ala His Leu Asp Ser Gly Pro Ser Thr Ala Lys Leu Asp Tyr
    370                 375                 380

Ser Lys Phe Gln Glu Leu Val Leu Pro His Phe Gly Lys Glu Asp Ala
385                 390                 395                 400

Gly Asp Val Gln Gln Phe Tyr Asp Leu Leu Ser Gly Ser Leu Glu Val
                405                 410                 415

Ile Arg Lys Trp Ala Glu Lys Ile Pro Gly Phe Ala Glu Leu Ser Pro
            420                 425                 430

Ala Asp Gln Asp Leu Leu Leu Glu Ser Ala Phe Leu Glu Leu Phe Ile
        435                 440                 445

Leu Arg Leu Ala Tyr Arg Ser Lys Pro Gly Glu Gly Lys Leu Ile Phe
    450                 455                 460

Cys Ser Gly Leu Val Leu His Arg Leu Gln Cys Ala Arg Gly Phe Gly
465                 470                 475                 480

Asp Trp Ile Asp Ser Ile Leu Ala Phe Ser Arg Ser Leu His Ser Leu
                485                 490                 495

Leu Val Asp Val Pro Ala Phe Ala Cys Leu Ser Ala Leu Val Leu Ile
            500                 505                 510

Thr Asp Arg His Gly Leu Gln Glu Pro Arg Arg Val Glu Glu Leu Gln
        515                 520                 525

Asn Arg Ile Ala Ser Cys Leu Lys Glu His Val Ala Ala Val Ala Gly
    530                 535                 540

Glu Pro Gln Pro Ala Ser Cys Leu Ser Arg Leu Leu Gly Lys Leu Pro
545                 550                 555                 560

Glu Leu Arg Thr Leu Cys Thr Gln Gly Leu Gln Arg Ile Phe Tyr Leu
                565                 570                 575

Lys Leu Glu Asp Leu Val Pro Pro Pro Pro Ile Ile Asp Lys Ile Phe
            580                 585                 590

Met Asp Thr Leu Pro Phe
        595

<210>  5
<211>  1572
<212>  DNA
<213>  Mus musculus

<220>
<221>  CDS
<222>  (8)..(1537)
<223>

<400>  5
aaacaag atg ccg tcg ggt ttc cag cag atc ggc tct gac gat ggg gaa        49
        Met Pro Ser Gly Phe Gln Gln Ile Gly Ser Asp Asp Gly Glu
        1               5                   10

ccc cct cgg cag cga gtg act gga aca ctg gtc cta gct gta ttc tca        97
Pro Pro Arg Gln Arg Val Thr Gly Thr Leu Val Leu Ala Val Phe Ser
15                  20                  25                  30

gct gtg ctt ggc tcc ctt cag ttt ggc tat aac att ggg gtt atc aat       145
Ala Val Leu Gly Ser Leu Gln Phe Gly Tyr Asn Ile Gly Val Ile Asn
                35                  40                  45

gcc cca cag aag gtg att gaa cag agc tac aat gca acg tgg ctg ggt       193
Ala Pro Gln Lys Val Ile Glu Gln Ser Tyr Asn Ala Thr Trp Leu Gly
            50                  55                  60

agg caa ggt cct ggg gga ccg gat tcc atc cca caa ggc acc ctc act 241
Arg Gln Gly Pro Gly Gly Pro Asp Ser Ile Pro Gln Gly Thr Leu Thr
        65                  70                  75

acg ctc tgg gct ctc tcc gtg gcc atc ttc tct gtg ggt ggc atg atc 289
Thr Leu Trp Ala Leu Ser Val Ala Ile Phe Ser Val Gly Gly Met Ile
    80                  85                  90

tct tcc ttt ctc att ggc atc att tct caa tgg ttg gga agg aaa agg 337
Ser Ser Phe Leu Ile Gly Ile Ile Ser Gln Trp Leu Gly Arg Lys Arg
95                  100                 105                 110

gct atg ctg gcc aac aat gtc ttg gcc gtg ttg ggg ggc gcc ctc atg 385
Ala Met Leu Ala Asn Asn Val Leu Ala Val Leu Gly Gly Ala Leu Met
                115                 120                 125

ggc cta gcc aat gcc gcg gcc tcc tat gag ata ctc att ctt gga cgg 433
Gly Leu Ala Asn Ala Ala Ala Ser Tyr Glu Ile Leu Ile Leu Gly Arg
            130                 135                 140

ttc ctc att ggc gcc tac tca ggg cta aca tca ggg ctg gtg ccc atg 481
Phe Leu Ile Gly Ala Tyr Ser Gly Leu Thr Ser Gly Leu Val Pro Met
        145                 150                 155

tat gtg gga gaa atc gcc ccc act cat ctt cgg ggt gcc ttg gga aca 529
Tyr Val Gly Glu Ile Ala Pro Thr His Leu Arg Gly Ala Leu Gly Thr
    160                 165                 170

ctc aac caa ctg gcc atc gtc att ggc att ctg gtt gcc cag gtg ctg 577
Leu Asn Gln Leu Ala Ile Val Ile Gly Ile Leu Val Ala Gln Val Leu
175                 180                 185                 190

ggc ttg gag tct atg ctg ggc aca gct acc ctg tgg cca ctg ctt ctg 625
Gly Leu Glu Ser Met Leu Gly Thr Ala Thr Leu Trp Pro Leu Leu Leu
                195                 200                 205

gct ctc aca gta ctc cct gct ctc ctg cag ctg att ctg ctg ccc ttc 673
Ala Leu Thr Val Leu Pro Ala Leu Leu Gln Leu Ile Leu Leu Pro Phe
            210                 215                 220

tgt cct gag agc ccc aga tac ctc tac atc atc cgg aac ctg gag ggg 721
Cys Pro Glu Ser Pro Arg Tyr Leu Tyr Ile Ile Arg Asn Leu Glu Gly
        225                 230                 235

cct gcc cga aag agt cta aag cgc ctg acc ggc tgg gct gat gtg tct 769
Pro Ala Arg Lys Ser Leu Lys Arg Leu Thr Gly Trp Ala Asp Val Ser
    240                 245                 250

gac gca cta gct gag ctg aag gat gag aaa cgg aag ttg gag aga gag 817
Asp Ala Leu Ala Glu Leu Lys Asp Glu Lys Arg Lys Leu Glu Arg Glu
255                 260                 265                 270

cgt cca atg tcc ttg ctc cag ctc ctg ggc agc cgc acc cac cgg cag 865
Arg Pro Met Ser Leu Leu Gln Leu Leu Gly Ser Arg Thr His Arg Gln
                275                 280                 285

cct ctg atc atc gca gtg gtg ctg cag ctg agc caa cag ctc tca ggc 913

Pro Leu Ile Ile Ala Val Val Leu Gln Leu Ser Gln Gln Leu Ser Gly
        290                 295                 300

atc aat gct gtt ttc tac tat tca acc agc atc ttc gag tcg gct ggg      961
Ile Asn Ala Val Phe Tyr Tyr Ser Thr Ser Ile Phe Glu Ser Ala Gly
    305                 310                 315

gtg gga cag cca gcc tac gcc acc ata gga gct ggt gtg gtc aat acg     1009
Val Gly Gln Pro Ala Tyr Ala Thr Ile Gly Ala Gly Val Val Asn Thr
    320                 325                 330

gtc ttc acg ttg gtc tcg gtg ctc tta gta gaa cga gct gga cga cgg     1057
Val Phe Thr Leu Val Ser Val Leu Leu Val Glu Arg Ala Gly Arg Arg
335                 340                 345                 350

aca ctc cat ctg ttg ggc ctg gcc ggc atg tgt ggc tgt gcc atc ttg     1105
Thr Leu His Leu Leu Gly Leu Ala Gly Met Cys Gly Cys Ala Ile Leu
                355                 360                 365

atg acc gtg gct ctg ctg ctg ctg gaa cgg gtt cca gcc atg agc tat     1153
Met Thr Val Ala Leu Leu Leu Leu Glu Arg Val Pro Ala Met Ser Tyr
            370                 375                 380

gtc tcc atc gtg gcc ata ttt ggc ttt gtg gcc ttc ttt gag att ggc     1201
Val Ser Ile Val Ala Ile Phe Gly Phe Val Ala Phe Phe Glu Ile Gly
        385                 390                 395

cct ggc ccc att ccc tgg ttc att gtg gca gag ctc ttc agc cag ggc     1249
Pro Gly Pro Ile Pro Trp Phe Ile Val Ala Glu Leu Phe Ser Gln Gly
    400                 405                 410

ccc cgc cca gcc gcc atg gct gtc gct ggt ttc tcc aac tgg acc tgt     1297
Pro Arg Pro Ala Ala Met Ala Val Ala Gly Phe Ser Asn Trp Thr Cys
415                 420                 425                 430

aac ttc att gtc ggc atg ggt ttc cag tat gtt gcg gat gct atg ggt     1345
Asn Phe Ile Val Gly Met Gly Phe Gln Tyr Val Ala Asp Ala Met Gly
                435                 440                 445

cct tac gtc ttc ctt cta ttt gcc gtc ctc ctg ctt ggc ttc ttc atc     1393
Pro Tyr Val Phe Leu Leu Phe Ala Val Leu Leu Leu Gly Phe Phe Ile
            450                 455                 460

ttc acc ttc cta aaa gtg cct gaa acc aga ggc cgg acg ttt gac cag     1441
Phe Thr Phe Leu Lys Val Pro Glu Thr Arg Gly Arg Thr Phe Asp Gln
        465                 470                 475

atc tca gct gcc ttc cga cgg aca cct tcc ctt tta gag cag gag gtg     1489
Ile Ser Ala Ala Phe Arg Arg Thr Pro Ser Leu Leu Glu Gln Glu Val
    480                 485                 490

aaa ccc agt aca gaa ctt gaa tac tta ggg cca gat gag aat gac tga     1537
Lys Pro Ser Thr Glu Leu Glu Tyr Leu Gly Pro Asp Glu Asn Asp
495                 500                 505

ggggcaaaac agggtgggag agccaccctc tccac                              1572

<210>  6
<211>  509
<212>  PRT
<213>  Mus musculus

<400>  6

Met Pro Ser Gly Phe Gln Gln Ile Gly Ser Asp Asp Gly Glu Pro Pro
1               5                   10                  15

Arg Gln Arg Val Thr Gly Thr Leu Val Leu Ala Val Phe Ser Ala Val
            20                  25                  30

Leu Gly Ser Leu Gln Phe Gly Tyr Asn Ile Gly Val Ile Asn Ala Pro
        35                  40                  45

Gln Lys Val Ile Glu Gln Ser Tyr Asn Ala Thr Trp Leu Gly Arg Gln
    50                  55                  60

Gly Pro Gly Gly Pro Asp Ser Ile Pro Gln Gly Thr Leu Thr Thr Leu
65                  70                  75                  80

Trp Ala Leu Ser Val Ala Ile Phe Ser Val Gly Gly Met Ile Ser Ser
                85                  90                  95

Phe Leu Ile Gly Ile Ile Ser Gln Trp Leu Gly Arg Lys Arg Ala Met
            100                 105                 110

Leu Ala Asn Asn Val Leu Ala Val Leu Gly Gly Ala Leu Met Gly Leu
        115                 120                 125

Ala Asn Ala Ala Ala Ser Tyr Glu Ile Leu Ile Leu Gly Arg Phe Leu
    130                 135                 140

Ile Gly Ala Tyr Ser Gly Leu Thr Ser Gly Leu Val Pro Met Tyr Val
145                 150                 155                 160

Gly Glu Ile Ala Pro Thr His Leu Arg Gly Ala Leu Gly Thr Leu Asn
                165                 170                 175

Gln Leu Ala Ile Val Ile Gly Ile Leu Val Ala Gln Val Leu Gly Leu
            180                 185                 190

Glu Ser Met Leu Gly Thr Ala Thr Leu Trp Pro Leu Leu Leu Ala Leu

195                 200                 205

Thr Val Leu Pro Ala Leu Leu Gln Leu Ile Leu Leu Pro Phe Cys Pro
    210                 215                 220

Glu Ser Pro Arg Tyr Leu Tyr Ile Ile Arg Asn Leu Glu Gly Pro Ala
225                 230                 235                 240

Arg Lys Ser Leu Lys Arg Leu Thr Gly Trp Ala Asp Val Ser Asp Ala
                245                 250                 255

Leu Ala Glu Leu Lys Asp Glu Lys Arg Lys Leu Glu Arg Glu Arg Pro
            260                 265                 270

Met Ser Leu Leu Gln Leu Leu Gly Ser Arg Thr His Arg Gln Pro Leu
        275                 280                 285

Ile Ile Ala Val Val Leu Gln Leu Ser Gln Gln Leu Ser Gly Ile Asn
    290                 295                 300

Ala Val Phe Tyr Tyr Ser Thr Ser Ile Phe Glu Ser Ala Gly Val Gly
305                 310                 315                 320

Gln Pro Ala Tyr Ala Thr Ile Gly Ala Gly Val Val Asn Thr Val Phe
                325                 330                 335

Thr Leu Val Ser Val Leu Leu Val Glu Arg Ala Gly Arg Arg Thr Leu
            340                 345                 350

His Leu Leu Gly Leu Ala Gly Met Cys Gly Cys Ala Ile Leu Met Thr
        355                 360                 365

Val Ala Leu Leu Leu Leu Glu Arg Val Pro Ala Met Ser Tyr Val Ser
    370                 375                 380

Ile Val Ala Ile Phe Gly Phe Val Ala Phe Phe Glu Ile Gly Pro Gly
385                 390                 395                 400

Pro Ile Pro Trp Phe Ile Val Ala Glu Leu Phe Ser Gln Gly Pro Arg
                405                 410                 415

Pro Ala Ala Met Ala Val Ala Gly Phe Ser Asn Trp Thr Cys Asn Phe
            420                 425                 430

Ile Val Gly Met Gly Phe Gln Tyr Val Ala Asp Ala Met Gly Pro Tyr
        435                 440                 445

Val Phe Leu Leu Phe Ala Val Leu Leu Leu Gly Phe Phe Ile Phe Thr
    450                 455                 460

Phe Leu Lys Val Pro Glu Thr Arg Gly Arg Thr Phe Asp Gln Ile Ser
465                 470                 475                 480

Ala Ala Phe Arg Arg Thr Pro Ser Leu Leu Glu Gln Glu Val Lys Pro
                485                 490                 495

Ser Thr Glu Leu Glu Tyr Leu Gly Pro Asp Glu Asn Asp
            500                 505

<210>  7
<211>  8402
<212>  DNA
<213>  Homo sapiens

<220>
<221>  CDS
<222>  (2225)..(2257)
<223>

<220>
<221>  CDS
<222>  (3535)..(3651)
<223>

<220>
<221>  CDS
<222>  (3763)..(3935)
<223>

<220>
<221>  CDS
<222>  (4028)..(4152)
<223>

<220>
<221>  CDS
<222>  (4276)..(4379)
<223>

<220>
<221> CDS
<222> (4507)..(4669)
<223>

<220>
<221> CDS
<222> (4775)..(4962)
<223>

<220>
<221> CDS
<222> (5125)..(5229)
<223>

<220>
<221> CDS
<222> (5378)..(5479)
<223>

<220>
<221> CDS
<222> (5995)..(6198)
<223>

<220>
<221> CDS
<222> (6716)..(6916)
<223>

<400> 7
gaattccgga tttttttttt tcttcttgag acggagtcac tctgtcgcca ggctggagtg 60

caagggcacg atcttggctc actacaacct ccacctcctg ggttcaagcc attttcctgc 120

ctcagcctcc cgagtagctg ggattacagg tgtgcataac cacgcccggc taatttttgt 180

atctttagca gacatggggt ttctctatgt tggccaggct ggtttcaaac tcctgacctc 240

agtcgatcca cctgccttgg cctcctaaag tgctgggatt acaggcatga gccaccaggc 300

cgggccggca ttccagattt ttcaggggat tcgtgcagca aaggaatcaa gaagggatgt 360

aaaggcacag tgtgttctgg gtacaataag gacttaggca ttgcccagaa caggaggcga 420

aggagataga aggagaggca ggagagatag gtaaggccag agatcggata agagaggcag 480

gaggttttgt tcactctgaa aagggatttg aacttggcaa ttggggcaac agagacagtg 540

acttcttgct tgagagatga gattggacct tcgaaaattg ttctctgccc tcgtcataaa 600

ggaaataaga ggagcacgaa gaccagtgag ggtgatggtg atctggactg aagtggcagc 660

cgccacggag aatatcggat gaatgtgaga gagttttgga ggtcaaagca ccaatgttgg 720

aaactaactg gataaacgag gagagcggcg caggacagga ggaatcgagc ctgacttcta 780

ccataggggt gactgggcgg gtaattcatt gaaataagga agttaggagg aggagcaggt 840

ttggacatgc tgatcactag agctgccaca tccgggcggt aacgaacacc tggatctgca 900

gctccagaga agggcctggg tcagatgtca ctgaagccct atggtggcgg aaaggcgaga 960

aatagtgggt tgagattcca agtgcaatcc actgcggctc ctcgctcgcc ctccaggtgg 1020

cagcacaacc ctgcgcttcc gaagcccgtt ttctgagcca gacactctcc acgctctggg 1080

tatttcggct tctctctccc cacacgccga ccctaggtcg cgcactttct gcctggcaga 1140

atttggccga gggatccaaa cccggagcag cctccagaga gcgtgtcgtt cacgcggcca 1200

gcatatgctc agagacctca gaggctcaga gacctcaggg ctggtggtgt ggtcggttgt 1260

gaccacttgt ccctcggacc ggctccagga accaacctgg ggaatgtgtg taggggaagg 1320

gcgggataga cagtgcccgg agcagggagg cgctgaaaga caggaccaag cagcccggcc 1380

accagacccg ttgtgggaac ggaatttcct ggcccccagg gccacactcg cgtgggaagc 1440

atgtcgcgga ccctttaagg cgtcatctcc ctgtctctcc gcccccgcct gggacaggcc 1500

gggacgcccg ggacctgaca tttggaggct cccaacgtgg gagctaaaaa tagcagcccc 1560

gggttacttt ggggcattgc tcctctccca acccgcgcgc cggctcgcga gccgtctcag 1620

gccgctggag tttccccggg gcaagtacac ctggcccgtc ctctcctctc agaccccact 1680

gtccagaccc gcagagttta agatgcttct gcagcccggg atcctagctg gtgggcggag 1740

tcctaacacg tgggtgggcg gggccttttg ttccagggac tcttttctca aaacttccca 1800

gtcggaggct ggcgggaacc cgagaggcgt gtctcgccag ccacgcggag gggcgtggcc 1860

tcattggccc gccccaccaa ctccagccaa actctaaacc ccaggcggag ggggcgtggc 1920

cttctggggt gtgcgggctc ctggccaatg ggtgctgtga agggcgtggc ccgcgggggc 1980

aggagcgagg tggcgggggc ttctcgcgtc ttttccccca gccccgctcc acaagatccg 2040

cgggagcccc actgctctcc ggatccttgg cttgtggctg tgggtcccat cgggcccgcc 2100

ctcgcacgtc actccgggac ccccgcggcc tccgcaggtt ctgcgctcca ggccggagtc 2160

agagactcca ggatcggttc tttcatcttc gccgcccctg cgcgtccagc tcttctaaga 2220

cgag atg ccg tcg ggc ttc caa cag ata ggc tcc gaa gtaggattca 2267
     Met Pro Ser Gly Phe Gln Gln Ile Gly Ser Glu
     1               5                   10

tcatgagggg gcggggcggg ggggcacggg tcccgctttt cttgggctgg ggtcgcggtt 2327

ggggtcagct gggggtggtt cctgcgcagg cgcagggggt gaaggtaggg ggctggctat 2387

ttatacccgg cctggacaac ccgtgactgt gagattccaa tcctaccaaa aggcagagtg 2447

ggtctggagg gcctttcggg gcacaggcag caagtggatt ctgcgagcca gggttcaccc 2507

ccttgcctag taggcggcgc ggcgctccgg aatcggggac accctgccct cgatccgact 2567

cgggaaagca gatccaggcg ggtcttgccc tccgggagct gtccgtccgt cttcgctcac 2627

gggcagtgtt tcgaggaccg gaggctctcc gtgggccccc acccccactc ctggccgccc 2687

tccaggacgc tgaactttcc cttggcccca tggttggtgg aggggcagag gggactgtca 2747

gccccccctc ccccagctca ggtttccgct tggagacagt ctgtgccgcc agcgagcggc 2807

caccactgcc accgcccctc acaccacctt cctgccctcc tcccctgggc atggctctcc 2867

caggcagaac ccctggacgg ccctccctgc acggaggtta gagggggagg gcaggccacg 2927

acgtagatag agaaggccac ccctagatga ccgggatgtc ctttctggaa cagcacttct 2987

tggtcctgtt gggggcctcc tggagctggc tgacagaacc cccagagggg agggaagagg 3047

acagtggctg atgataataa tgcacgtgtt aatttatgaa accagcactg tcaaggatat 3107

tgttaacatg tgatgttgat tttcacaaca ctctcacaga taggtaggca aggcaggcaa 3167

catcaccccc atctcacaga ggacactcag gttcggggca gggaagtgac ttggccaagg 3227

tcacacaaat ctgagctctt aaggccaagc ctgtctcaag gtcacagaag aattttgaga 3287

caagttctca aacatttctc tgccttatgg acccaaacat ccagtttctc ctttatgccc 3347

aggttgcagt tcagctcctg tttacattga gatctttgtg caattcctaa tatggcccag 3407

tttccctcac ccaacatgtt gggtggagcc cagtatcttc aggctccagc tgggcccggg 3467

cccctagcgg aaggaaaaaa atcatggttc catgtgacat gctgtgtctt tgtgtctgcc 3527

tgttcag gat ggg gaa ccc cct cag cag cga gtg act ggg acc ctg gtc 3576
Asp Gly Glu Pro Pro Gln Gln Arg Val Thr Gly Thr Leu Val
            15                  20                  25

ctt gct gtg ttc tct gcg gtg ctt ggc tcc ctg cag ttt ggg tac aac 3624
Leu Ala Val Phe Ser Ala Val Leu Gly Ser Leu Gln Phe Gly Tyr Asn
                30                  35                  40

att ggg gtc atc aat gcc cct cag aag gtgagggcct gcagctggca 3671
Ile Gly Val Ile Asn Ala Pro Gln Lys
            45                  50

gggtgggggt acccaaacga ggaggacagg tgtctcgggg gtggtggaaa ggggacggtc 3731

tgcaggaaat ctgtcctctg ctgtccccca g gtg att gaa cag agc tac aat 3783
Val Ile Glu Gln Ser Tyr Asn
                55

gag acg tgg ctg ggg agg cag ggg cct gag gga ccc agc tcc atc cct 3831
Glu Thr Trp Leu Gly Arg Gln Gly Pro Glu Gly Pro Ser Ser Ile Pro
        60                  65                  70

cca ggc acc ctc acc acc ctc tgg gcc ctc tcc gtg gcc atc ttt tcc 3879
Pro Gly Thr Leu Thr Thr Leu Trp Ala Leu Ser Val Ala Ile Phe Ser
    75                  80                  85

gtg ggc ggc atg att tcc tcc ttc ctc att ggt atc atc tct cag tgg 3927
Val Gly Gly Met Ile Ser Ser Phe Leu Ile Gly Ile Ile Ser Gln Trp
90                  95                  100                 105

ctt gga ag gttcgcagct ggagggcagg ggtgggggaa acaggaaggg 3975
Leu Gly Arg

agccactgct gggtgccctc accctcacag cctcactctg tctgcctgcc ag g aaa 4031
Lys

agg gcc atg ctg gtc aac aat gtc ctg gcg gtg ctg ggg ggc agc ctc 4079
Arg Ala Met Leu Val Asn Asn Val Leu Ala Val Leu Gly Gly Ser Leu
110                 115                 120                 125

atg ggc ctg gcc aac gct gct gcc tcc tat gaa atg ctc atc ctt gga 4127
Met Gly Leu Ala Asn Ala Ala Ala Ser Tyr Glu Met Leu Ile Leu Gly
                130                 135                 140

cga ttc ctc att ggc gcc tac tca g gtactcacgg gcaccacagc 4172
Arg Phe Leu Ile Gly Ala Tyr Ser
            145

cctgcctagc gccctgttct ctttcaccat gcctgggctt tcagatggga atggacacct 4232

gccctcagcc ctctcttctt ccctcgccca gggctgacat cag gg ctg gtg ccc 4286
Gly Leu Val Pro

atg tac gtg ggg gag att gct ccc act cac ctg cgg ggc gcc ctg ggg 4334
Met Tyr Val Gly Glu Ile Ala Pro Thr His Leu Arg Gly Ala Leu Gly
    155                 160                 165

acg ctc aac caa ctg gcc att gtt atc ggc att ctg atc gcc cag 4379
Thr Leu Asn Gln Leu Ala Ile Val Ile Gly Ile Leu Ile Ala Gln
170                 175                 180

gtgaccggag caagcctcat gggtgcctgg gcagtggtta gagtggggct ctggagaata 4439

tggtgggctt ccaaggtaag gcagaagggc tgagtgacct gccttctttc ccaaccttct 4499

cccacag gtg ctg ggc ttg gag tcc ctc ctg ggc act gcc agc ctg tgg 4548
Val Leu Gly Leu Glu Ser Leu Leu Gly Thr Ala Ser Leu Trp
185                 190                 195

cca ctg ctc ctg ggc ctc aca gtg cta cct gcc ctc ctg cag ctg gtc     4596
Pro Leu Leu Leu Gly Leu Thr Val Leu Pro Ala Leu Leu Gln Leu Val
    200                 205                 210

ctg ctg ccc ttc tgt ccc gag agc ccc cgc tac ctc tac atc atc cag     4644
Leu Leu Pro Phe Cys Pro Glu Ser Pro Arg Tyr Leu Tyr Ile Ile Gln
215                 220                 225                 230

aat ctc gag ggg cct gcc aga aag a gtaagctctc ccgctgcagc             4689
Asn Leu Glu Gly Pro Ala Arg Lys
                235

ctggcccagg cccatgcctc cgcctcatct tgctagcacc tggcttcctc tcaggtcccc   4749

tcaggcctga ccttcccttc tccag gt  ctg aag cgc ctg aca ggc tgg gcc     4800
                            Ser Leu Lys Arg Leu Thr Gly Trp Ala
                                    240                 245

gat gtt tct gga gtg ctg gct gag ctg aag gat gag aag cgg aag ctg     4848
Asp Val Ser Gly Val Leu Ala Glu Leu Lys Asp Glu Lys Arg Lys Leu
        250                 255                 260

gag cgt gag cgg cca ctg tcc ctg ctc cag ctc ctg ggc agc cgt acc     4896
Glu Arg Glu Arg Pro Leu Ser Leu Leu Gln Leu Leu Gly Ser Arg Thr
    265                 270                 275

cac cgg cag ccc ctg atc att gcg gtc gtg ctg cag ctg agc cag cag     4944
His Arg Gln Pro Leu Ile Ile Ala Val Val Leu Gln Leu Ser Gln Gln
280                 285                 290                 295

ctc tct ggc atc aat gct gtatgtgtgg agcagcctcc aggcagggca            4992
Leu Ser Gly Ile Asn Ala
                300

cagccccggg agggtagacg agagtgggga gcaaaccccc tccaccaaca cccagggtag   5052

ggccagcctg ttgtggctgg agtagaggaa ggggcattcc tgccatcact tcttcttctc   5112

ccccacctct ag gtt ttc tat tat tcg acc agc atc ttc gag aca gca ggg   5163
              Val Phe Tyr Tyr Ser Thr Ser Ile Phe Glu Thr Ala Gly
                          305                 310

gta ggc cag cct gcc tat gcc acc ata gga gct ggt gtg gtc aac aca     5211
Val Gly Gln Pro Ala Tyr Ala Thr Ile Gly Ala Gly Val Val Asn Thr
315                 320                 325                 330

gtc ttc acc ttg gtc tcg gtaactgctc acctctggaa tggcccgagc            5259
Val Phe Thr Leu Val Ser
                335

cactggcttc acctccctgg gtgtcccgga ggtcctgctc ttggttgccc tcacccacgc   5319

ggcccctcct acttcccgtg cccaaaaggc tggggtcaag ctccgactct ccccgcag      5377

gtg ttg ttg gtg gag cgg gcg ggg cgc cgg acg ctc cat ctc ctg ggc     5425
Val Leu Leu Val Glu Arg Ala Gly Arg Arg Thr Leu His Leu Leu Gly

340                 345                 350

ctg gcg ggc atg tgt ggc tgt gcc atc ctg atg act gtg gct ctg ctc     5473
Leu Ala Gly Met Cys Gly Cys Ala Ile Leu Met Thr Val Ala Leu Leu
        355                 360                 365

ctg ctg gtaaggcctg gaggctagga ggggctagca gcccacccca tgggaatggt     5529
Leu Leu
    370

cctgtgagtc tctgtgacca gccagggtcc cttcttaaca cacatgcttt caatcctggc     5589

gccagctccg gaccaggact ggggctgact ggctccagaa tctgctggga ttgtggtctg     5649

ctcctgaggg atttgggctg cactgggagg cagctgtggc acaactctgg cagccaggag     5709

ggagagcccc tgtcaagcct caggaacaat cattcctaag gacccagctt tagagtccag     5769

ggagagctga ccgtcataag aactgagagg ccataacatt tcctctgcct tgaacccact     5829

tgggatagcc agcagaatgc cagtcaaggg cctgctctaa cccgggacag caggccccct     5889

acaagctgct gcggaggggg ttaggtttca cttctctgag ttgagggcaa gggaagatca     5949

gaaaggcctc aactggattc tccaccctcc ctgtctggcc cctag gag cga gtt cca     6006
                                                  Glu Arg Val Pro

gcc atg agc tac gtc tcc att gtg gcc atc ttt ggc ttc gtg gca ttt     6054
Ala Met Ser Tyr Val Ser Ile Val Ala Ile Phe Gly Phe Val Ala Phe
375                 380                 385                 390

ttt gag att ggc cct ggc ccc att cct tgg ttc atc gtg gcc gag ctc     6102
Phe Glu Ile Gly Pro Gly Pro Ile Pro Trp Phe Ile Val Ala Glu Leu
                395                 400                 405

ttc agc cag gga ccc cgc ccg gca gcc atg gct gtg gct ggt ttc tcc     6150
Phe Ser Gln Gly Pro Arg Pro Ala Ala Met Ala Val Ala Gly Phe Ser
            410                 415                 420

aac tgg acg agc aac ttc atc att ggc atg ggt ttc cag tat gtt gcg     6198
Asn Trp Thr Ser Asn Phe Ile Ile Gly Met Gly Phe Gln Tyr Val Ala
        425                 430                 435

gtaggtcccc ccgccccagc ctcccacacc gtaggccaga ggtgggcatc acacagctag     6258

cccacctgct tccccgtcag ggactcctcc agccacagac catgggtctt tgggtcagtt     6318

tggtggacca cctgctccac agaatcaaag caaggaaggg agctgaccta gattggatag     6378

taactgaggt gtctgaaacg caccagtggc ataacttacc ttactccaag aataaaatga     6438

tacactttgc attaatacta caaacagctg ggactctcct ctgagtgcag taactgagga     6498

tggtgaagag ggcgaaaact aagagtgttt ggggttcaga gaatcctctt ttcagtgtaa     6558

attctcattc ctgctcattt cccttgtccc tggaggaggc agctgctgtc tgccgtcccc     6618

ccagctccct atgaaggcct ttagctcctg gttgcctgaa actacccctt ccctccccac 6678

ctcactccgt caacacctct ttctccacct gtcccag gag gct atg ggg ccc tac 6733
Glu Ala Met Gly Pro Tyr
    440

gtc ttc ctt cta ttt gcg gtc ctc ctg ctg ggc ttc ttc atc ttc acc 6781
Val Phe Leu Leu Phe Ala Val Leu Leu Leu Gly Phe Phe Ile Phe Thr
445                 450                 455                 460

ttc tta aga gta cct gaa act cga ggc cgg acg ttt gac cag atc tca 6829
Phe Leu Arg Val Pro Glu Thr Arg Gly Arg Thr Phe Asp Gln Ile Ser
                465                 470                 475

gct gcc ttc cac cgg aca ccc tct ctt tta gag cag gag gtg aaa ccc 6877
Ala Ala Phe His Arg Thr Pro Ser Leu Leu Glu Gln Glu Val Lys Pro
            480                 485                 490

agc aca gaa ctt gag tat tta ggg cca gat gag aac gac tgaggggcca 6926
Ser Thr Glu Leu Glu Tyr Leu Gly Pro Asp Glu Asn Asp
        495                 500                 505

ggcaggggtg ggagagccag ctctctctac ccggcccaga gaccccttcc tttcctctgc 6986

agcactttaa ccctctcttc cctattattt ccgggtggaa aagaatccct gcagcctggt 7046

agaattggga agctggggga agggtggtct gagcaccccc tcattcccct cgtgtgactc 7106

tcttggatta tttatgtgtt gtggtttggc cgtggccatc agggtgggcc actctcccct 7166

ccctcttcct tcccccatcc cctttcctcc ccaccttccc cagactcagc tccagaatac 7226

cttcttcgct gctagagaag ggggattgga gggaagacag gtctagactt tctcagtggg 7286

acaaaccaga gcagagagca ggacaggaga caagaaatcc agtttcccac caccttggac 7346

tcctcccaca atctgggact ttcactgaat tcttgccacg cagactctgg gcaaaggggg 7406

ttctcttttt tttttttttt ttttttttga gacagtctcg ctctgtcgcc caggctcgag 7466

tgcagtggcg tgatcttgct tcactgcaag ctgtctccca ggttcacgcc attctcctgc 7526

ctcagcctcc ggagtagctg ggactacagg cgcatgccac cacacctggc taatttattt 7586

tgtattttta gtatatacgc ggtttcacca tgttagccag aatggtctcg atctcctgac 7646

ctcgtgatct gcctgcctca gcctcccaaa gtgctgggat tacaggcgtg agccaccgcg 7706

cctggcgaag ggagttctct cttcgacccc tgcaggctca gccttccagg gcaagaggga 7766

acaggaaagt atgtgcccat gtgtggcaag atggaaggac ggcaggctcc cgcctctagg 7826

cttggggctc taccccgatg gtttcccaag gctgccaaga aggagcccta actttcttcc 7886

tctcccttcc tggaagggtg ctgcatccac aggcttttga ccaactaagg caaagagggg 7946

atttgaaagg ctgcctggaa acactgggct gggaggagcc tttggatatt tttatatacg 8006

tttgaaaagg ggattgagag aagaaaccaa aggtcggttg tactaaatgt atatatatag 8066

atacttctat aaagtcactg ctgaagacaa gcatcctatt gtggaggtac ttgaggatgg 8126

gctgagacag ggaccataac tcttcacccc tcttcctccc tctgtcctgc ctcagctcaa 8186

ggcctcagaa tcttctggat gccattgctc atgcccctac tcacatttct actcgttgct 8246

ttattaatag taaatgctca ataaattgta gctgccagtg ccgggcattg ctcttggcat 8306

ttgcagaata ctcactctgt gagggaggtg tcagcccatg tcacagatgg gcagtgaaac 8366

ccatgatagg ggcactcttc accagggaca cagctg 8402

<210> 8
<211> 505
<212> PRT
<213> Homo sapiens

<400> 8

Met Pro Ser Gly Phe Gln Gln Ile Gly Ser Glu Asp Gly Glu Pro Pro
1               5                   10                  15

Gln Gln Arg Val Thr Gly Thr Leu Val Leu Ala Val Phe Ser Ala Val
            20                  25                  30

Leu Gly Ser Leu Gln Phe Gly Tyr Asn Ile Gly Val Ile Asn Ala Pro
        35                  40                  45

Gln Lys Val Ile Glu Gln Ser Tyr Asn Glu Thr Trp Leu Gly Arg Gln
    50                  55                  60

Gly Pro Glu Gly Pro Ser Ser Ile Pro Pro Gly Thr Leu Thr Thr Leu
65                  70                  75                  80

Trp Ala Leu Ser Val Ala Ile Phe Ser Val Gly Gly Met Ile Ser Ser
                85                  90                  95

Phe Leu Ile Gly Ile Ile Ser Gln Trp Leu Gly Arg Lys Arg Ala Met
            100                 105                 110

Leu Val Asn Asn Val Leu Ala Val Leu Gly Gly Ser Leu Met Gly Leu
        115                 120                 125

Ala Asn Ala Ala Ala Ser Tyr Glu Met Leu Ile Leu Gly Arg Phe Leu

130                 135                 140

Ile Gly Ala Tyr Ser Gly Leu Val Pro Met Tyr Val Gly Glu Ile Ala
145                 150                 155                 160

Pro Thr His Leu Arg Gly Ala Leu Gly Thr Leu Asn Gln Leu Ala Ile
                165                 170                 175

Val Ile Gly Ile Leu Ile Ala Gln Val Leu Gly Leu Glu Ser Leu Leu
            180                 185                 190

Gly Thr Ala Ser Leu Trp Pro Leu Leu Leu Gly Leu Thr Val Leu Pro
        195                 200                 205

Ala Leu Leu Gln Leu Val Leu Leu Pro Phe Cys Pro Glu Ser Pro Arg
    210                 215                 220

Tyr Leu Tyr Ile Ile Gln Asn Leu Glu Gly Pro Ala Arg Lys Ser Leu
225                 230                 235                 240

Lys Arg Leu Thr Gly Trp Ala Asp Val Ser Gly Val Leu Ala Glu Leu
                245                 250                 255

Lys Asp Glu Lys Arg Lys Leu Glu Arg Glu Arg Pro Leu Ser Leu Leu
            260                 265                 270

Gln Leu Leu Gly Ser Arg Thr His Arg Gln Pro Leu Ile Ile Ala Val
        275                 280                 285

Val Leu Gln Leu Ser Gln Gln Leu Ser Gly Ile Asn Ala Val Phe Tyr
    290                 295                 300

Tyr Ser Thr Ser Ile Phe Glu Thr Ala Gly Val Gly Gln Pro Ala Tyr
305                 310                 315                 320

Ala Thr Ile Gly Ala Gly Val Val Asn Thr Val Phe Thr Leu Val Ser
                325                 330                 335

Val Leu Leu Val Glu Arg Ala Gly Arg Arg Thr Leu His Leu Leu Gly
            340                 345                 350

Leu Ala Gly Met Cys Gly Cys Ala Ile Leu Met Thr Val Ala Leu Leu
        355                 360                 365

Leu Leu Glu Arg Val Pro Ala Met Ser Tyr Val Ser Ile Val Ala Ile
    370                 375                 380

Phe Gly Phe Val Ala Phe Phe Glu Ile Gly Pro Gly Pro Ile Pro Trp
385                 390                 395                 400

Phe Ile Val Ala Glu Leu Phe Ser Gln Gly Pro Arg Pro Ala Ala Met
                405                 410                 415

Ala Val Ala Gly Phe Ser Asn Trp Thr Ser Asn Phe Ile Ile Gly Met
            420                 425                 430

Gly Phe Gln Tyr Val Ala Glu Ala Met Gly Pro Tyr Val Phe Leu Leu
        435                 440                 445

Phe Ala Val Leu Leu Leu Gly Phe Phe Ile Phe Thr Phe Leu Arg Val
    450                 455                 460

Pro Glu Thr Arg Gly Arg Thr Phe Asp Gln Ile Ser Ala Ala Phe His
465                 470                 475                 480

Arg Thr Pro Ser Leu Leu Glu Gln Glu Val Lys Pro Ser Thr Glu Leu
                485                 490                 495

Glu Tyr Leu Gly Pro Asp Glu Asn Asp
            500                 505